# EUROPEAN PATENT SPECIFICATION

(11) **EP 0 462 830 B1**
(45) Date of publication and mention of the grant of the patent: **18.10.1995**
(21) Application number: 91305575.2
(22) Date of filing: 19.06.1991
(51) Int. Cl.: C07D 309/04, C07D 405/12, C07D 309/08, A61K 31/35, A61K 31/47

(54) **Cyclic ether derivatives**
Zyklische Ether-Derivate
Dérivés cycliques d'éther

(30) Priority: 21.06.1990 EP 90401755
(43) Date of publication of application: 27.12.1991
(73) Proprietor: ZENECA LIMITED, London W1Y 6LN (GB); ZENECA Pharma S.A., 95022 Cergy Cédex (FR)
(72) Inventor: Bruneau, Pierre Andre Raymond, F-51500 Ludes (FR); Dowell, Robert Ian, Congleton, Cheshire CW12 3DD (GB); Waterson, David, Macclesfield, Cheshire SK10 5NX (GB)
(74) Representative: Tait, Brian Steele

(56) References cited:
- EP-A- 0 375 404
- EP-A- 0 385 662
- FR-A- 1 382 753
- GB-A- 1 060 005
- US-A- 2 242 575

## Description

This invention concerns novel cyclic ether derivatives and more particularly novel cyclic ether derivatives which are inhibitors of the enzyme 5-lipoxygenase (hereinafter referred to as 5-LO). The invention also concerns processes for the manufacture of said cyclic ether derivatives and novel pharmaceutical compositions containing them. Also included in the invention is the use of said cyclic ether derivatives in the treatment of various inflammatory and/or allergic diseases in which the direct or indirect products of 5-LO catalysed oxidation of arachidonic acid are involved, and the production of new medicaments for such use.

As stated above the cyclic ether derivatives described hereinafter are inhibitors of 5-LO, which enzyme is known to be involved in catalysing the oxidation of arachidonic acid to give rise via a cascade process to the physiologically active leukotrienes such as leukotriene B₄ (LTB₄) and the peptido-lipid leukotrienes such as leukotriene C₄ (LTC₄) and leukotriene D₄ (LTD₄) and various metabolites.

The biosynthetic relationship and physiological properties of the leukotrienes are summarised by G.W. Taylor and S.R. Clarke in Trends in Pharmacological Sciences, 1986, 7, 100-103. The leukotrienes and their metabolites have been implicated in the production and development of various inflammatory and allergic diseases such as arthritic diseases, asthma, allergic rhinitis, atopic dermatitis, psoriasis, cardiovascular and cerebrovascular disorders and inflammatory bowel disease. In addition the leukotrienes are mediators of inflammatory diseases by virtue of their ability to modulate lymphocyte and leukocyte function. Other physiologically active metabolites of arachidonic acid, such as the prostaglandins and thromboxanes, arise via the action of the enzyme cyclooxygenase on arachidonic acid.

We have now discovered that certain cyclic ether derivatives are effective as inhibitors of the enzyme 5-LO and thus of leukotriene biosyntheses. Thus, such compounds are of value as therapeutic agents in the treatment of, for example, allergic conditions, psoriasis, asthma, cardiovascular and cerebrovascular disorders, and/or inflammatory and arthritic conditions, mediated alone or in part by one or more leukotrienes.

According to the invention there is provided a cyclic ether derivative of the formula I (set out hereinafter)
wherein Ar¹ is phenyl or naphthyl, or a 9- or 10-membered bicyclic heterocyclic moiety containing one or two nitrogen heteroatoms and optionally containing a further heteroatom selected from nitrogen, oxygen and sulphur, and Ar¹ may optionally bear up to five substituents selected from amino, halogeno, hydroxy, cyano, oxo, thioxo, (1-4C)alkyl, (1-4C)alkoxy, (1-4C)alkylthio, (1-4C)alkylsulphinyl, (1-4C)alkylsulphonyl, (1-4C)alkylamino, di-[(1-4C)alkyl]amino, (2-4C)alkanoyl, fluoro-(1-4C)alkyl, cyano-(1-4C)alkyl, phenyl, benzoyl and phenyl-(1-4C)alkyl, and wherein said phenyl, benzoyl or phenyl-(1-4C)alkyl substituents may optionally bear a substituent selected from halogeno, (1-4C)alkyl and (1-4C)alkoxy;
wherein A¹ is a direct link to X¹ or is (1-3C)alkylene;
wherein X¹ is oxy, thio, sulphinyl, sulphonyl or imino;
wherein Ar² is phenylene which may optionally bear one or two substituents selected from halogeno, hydroxy, amino, nitro, cyano carbamoyl, ureido, (1-4C)alkyl, (1-4C)alkoxy, (1-4C)alkylamino, di-[(1-4C)alkyl]amino, fluoro-(1-4C)alkyl and (2-4C)alkanoylamino; or Ar² is pyridylene;
wherein R¹ is (1-4C)alkylthio, (1-4C)alkylsulphinyl, (1-4C)alkylsulphonyl, (1-4C)alkylamino or di-[(1-4C)alkyl]amino, or R¹ is hydrogen, formyl, cyano, carbamoyl, (1-4C)alkyl, (1-4C)alkoxycarbonyl, N-(1-4C)alkylcarbamoyl, N,N-di-[(1-4C)alkyl]carbamoyl, hydroxy-(1-4C)alkyl, fluoro-(1-4C)alkylthio, (2-4C)alkanoyl or (1-4C)alkoxy-(1-4C)alkyl; and
wherein R² and R³ together form a group of the formula -A²-X²-A³- which, together with the carbon atom to which A² and A³ are attached, defines a ring having 5 to 7 ring atoms, wherein A² and A³, which may be the same or different, each is (1-3C)alkylene and X² is oxy, thio, sulphinyl or sulphonyl, and which ring may bear one, two or three substituents, which may be the same or different, selected from hydroxy, (1-4C)alkyl and (1-4C)alkoxy;
or a pharmaceutically-acceptable salt thereof.

The chemical formulae referred to herein by Roman numerals are set out for convenience on a separate sheet hereinafter.

In this specification the generic term "alkyl" includes both straight-chain and branched-chain alkyl groups. However references to individual alkyl groups such as "propyl" are specific for the straight-chain version only and references to individual branched-chain alkyl groups such as "isopropyl" are specific for the branched-chain version only. An analogous convention applies to other generic terms.

It is to be understood that, insofar as certain of the compounds of the formula I defined above may exhibit the phenomenon of tautomerism and any formula drawing presented herein may represent only one of the possible tautomeric forms, the invention includes in its definition any tautomeric form of a compound of the formula I which possesses the property of inhibiting 5-LO and is not to be limited merely to any one tautomeric form utilised within the formulae drawings.

It is further to be understood that, insofar as certain of the compounds of formula I defined above may exist in optically active or racemic forms by virtue of one or more substituents containing an asymmetric carbon atom, the invention includes in its definition any such optically active or racemic form which possesses the property of inhibiting 5-LO. The synthesis of optically active forms may be carried out by standard techniques of organic chemistry well known in the art, for example by synthesis from optically active starting materials or by resolution of a racemic form. Similarly, inhibitory properties against 5-LO may be evaluated using the standard laboratory techniques refereed to hereinafter.

According to a further feature of the invention there is provided a cyclic ether derivative of the formula I wherein Ar¹ is phenyl or naphthyl, or a 9- or 10-membered bicyclic heterocyclic moiety containing one or two nitrogen heteroatoms and optionally containing a further heteroatom selected from nitrogen, oxygen and sulphur, and Ar¹ may optionally bear up to five substituents selected from amino, halogeno, hydroxy, cyano, oxo, thioxo, (1-4C)alkyl, (1-4C)alkoxy, (1-4C)alkylthio, (1-4C)alkylsulphinyl, (1-4C)alkylsulphonyl, (1-4C)alkylamino, di-[(1-4C)alkyl]amino, (2-4C)alkanoyl, fluoro-(1-4C)alkyl, cyano-(1-4C)alkyl, phenyl, benzoyl and phenyl-(1-4C)alkyl, and wherein said phenyl, benzoyl or phenyl-(1-4C)alkyl substituents may optionally bear a substituent selected from halogeno, (1-4C)alkyl and (1-4C)alkoxy;
wherein A¹ is a direct link to X¹ or is (1-3C)alkylene;
wherein X¹ is oxy, thio, sulphinyl, sulphonyl or imino;
wherein Ar² is phenylene which may optionally bear one or two substituents selected from halogeno, hydroxy, amino, nitro, cyano carbamoyl, ureido, (1-4C)alkyl, (1-4C)alkoxy, (1-4C)alkylamino, di-[(1-4C)alkyl]amino, fluoro-(1-4C)alkyl and (2-4C)alkanoylamino; or Ar² is pyridylene;
wherein R¹ is (1-4C)alkylthio, (1-4C)alkylsulphinyl, (1-4C)alkylsulphonyl, (1-4C)alkylamino or di-[(1-4C)alkyl]amino, or R¹ is cyano, carbamoyl, (1-4C)alkoxycarbonyl, N-(1-4C)alkylcarbamoyl, N,N-di-[(1-4C)alkyl]carbamoyl, hydroxy-(1-4C)alkyl or (1-4C)alkoxy-(1-4C)alkyl; and
wherein R² and R³ together form a group of the formula -A²-X²-A³- which, together with the carbon atom to which A² and A³ are attached, defines a ring having 5 to 7 ring atoms, wherein A² and A³, which may be the same or different, each is (1-3C)alkylene and X² is oxy, thio, sulphinyl or sulphonyl, and which ring may bear one, two or three substituents, which may be the same or different, selected from hydroxy, (1-4C)alkyl and (1-4C)alkoxy;
or a pharmaceutically-acceptable salt thereof.

Suitable values for the generic terms referred to above include those set out below.

A suitable value for Ar¹ when it is a 9- or 10-membered bicyclic heterocyclic moiety containing one or two nitrogen heteroatoms and optionally containing a further heteroatom selected from nitrogen, oxygen and sulphur is, for example, a 9- or 10-membered benzo-fused heterocyclic moiety such as indolyl, isoindolyl, benzimidazolyl, 1H-indazolyl, benzoxazolyl, benzothiazolyl, quinolyl, isoquinolyl, cinnolinyl, quinazolinyl, quinoxalinyl, 4H-1,4-benzoxazinyl or 4H-1,4-benzothiazinyl, or a hydrogenated derivative thereof such as indolinyl, 2,3-dihydrobenzimidazolyl, 2,3-dihydrobenzoxazolyl, 2,3-dihydrobenzothiazolyl, 1,2-dihydroquinolyl, 1,2,3,4-tetrahydroquinolyl, 1,2-dihydroisoquinolyl, 2,3-dihydro-4H-1,4-benzoxazinyl or 2,3-dihydro-4H-1,4-benzothiazinyl; or, for example, a 9- or 10-membered pyrido-fused heterocyclic moeity such as 1H-pyrrolo[2,3-b]pyridyl, imidazo[4,5-b]pyridyl, 1,7-naphthyridinyl, 1,8-naphthyridinyl, pyrido[2,3-d]pyrimidinyl, pyrido[2,3-b]pyrazinyl, 4H-pyrido[3,2-b][1,4]oxazinyl and 4H-pyrido[3,2-b][1,4]thiazinyl, or a hydrogenated derivative thereof.

The heterocyclic moiety may be attached through any available position including from either of the two rings of the bicyclic heterocyclic moiety and including through an available nitrogen atom. The heterocyclic moiety may bear a suitable substituent such as, for example, a (1-4C)alkyl, phenyl, benzoyl or phenyl-(1-4C)alkyl substituent on an available nitrogen atom.

Suitable values for substituents which may be present on Ar¹ or Ar², or on a phenyl, benzoyl or phenyl-(1-4C)alkyl substituent on Ar¹, include, for example:-
- for halogeno:: fluoro, chloro, bromo and iodo;
- for (1-4C)alkyl:: methyl, ethyl, propyl, isopropyl, butyl, isobutyl, sec-butyl and tert-butyl;
- for (1-4C)alkoxy:: methoxy, ethoxy, propoxy, isopropoxy and butoxy;
- for fluoro-(1-4C)alkyl:: fluoromethyl, difluoromethyl, trifluoromethyl, 2-fluoroethyl, 2,2,2-trifluoroethyl and pentafluoroethyl;
- for (1-4C)alkylamino:: methylamino, ethylamino, propylamino and butylamino;
- for di-[(1-4C)alkyl]amino:: dimethylamino, diethylamino and dipropylamino.

Suitable values for substituents which may be present on Ar¹ include, for example:-
- for (1-4C)alkythio:: methylthio, ethylthio, propylthio, isopropylthio and butylthio;
- for (1-4C)alkylsulphinyl:: methylsulphinyl, ethylsulphinyl, propylsulphinyl, isopropylsulphinyl and butylsulphinyl;
- for (1-4C)alkylsulphonyl:: methylsulphonyl, ethylsulphonyl, propylsulphonyl, isopropylsulphonyl and butylsulphonyl;
- for (2-4C)alkanoyl:: acetyl, propionyl and butyryl;
- for cyano-(1-4C)alkyl:: cyanomethyl, 1-cyanoethyl, 2-cyanoethyl, 3-cyanopropyl and 2-cyanoprop-2-yl;
- for phenyl-(1-4C)alkyl:: benzyl, phenethyl and 3-phenylpropyl.

A suitable value for A¹ when it is (1-3C)alkylene is, for example, methylene, ethylene or trimethylene.

A suitable value for Ar² when it is phenylene is, for example 1,3-phenylene or 1,4-phenylene.

A suitable value for Ar² when it is pyridylene is, for example 3,5- or 2,6-pyridylene.

A suitable value for a (2-4C)alkanoylamino substituent which may be present on Ar² is, for example, acetamido, propionamido or butyramido.

A suitable value for R¹ when it is (1-4C)alkylthio is, for example, methylthio, ethylthio, propylthio, isopropylthio or butylthio; when it is (1-4C)alkylsulphinyl is, for example, methylsulphinyl, ethylsulphinyl, propylsulphinyl, isopropylsulphinyl or butylsulphinyl; when it is (1-4C)alkylsulphonyl is, for example, methylsulphonyl, ethylsulphonyl, propylsulphonyl, isopropylsulphonyl and butylsulphonyl; when it is (1-4C)alkylamino is, for example, methylamino, ethylamino, propylamino, isopropylamino or butylamino; and when it is di-[(1-4C)alkyl]amino is, for example, dimethylamino, diethylamino or dipropylamino.

A suitable value for R¹ when it is (1-4C)alkyl is, for example, methyl, ethyl, propyl, isopropyl, butyl or isobutyl; when it is (1-4C)alkoxycarbonyl is, for example, methoxycarbonyl, ethoxycarbonyl, propoxycarbonyl, isopropoxycarbonyl or butoxycarbonyl; when it is N-(1-4C)alkylcarbamoyl is, for example, N-methylcarbamoyl, N-ethylcarbamoyl or N-propylcarbamoyl; when it is N,N-di-[(1-4C)alkyl]carbamoyl is, for example, N,N-dimethylcarbamoyl, N,N-diethylcarbamoyl or N,N-dipropylcarbamoyl; when it is hydroxy-(1-4C)alkyl is, for example, hydroxymethyl, 1-hydroxyethyl, 2-hydroxyethyl, 2-hydroxypropyl or 3-hydroxypropyl; when it is fluoro-(1-4C)alkylthio is, for example, fluoromethylthio, difluoromethylthio, trifluoromethylthio, 2-fluoroethylthio, 2,2,2-trifluoroethylthio or pentafluoroethylthio; when it is (2-4C)alkanoyl is, for example, acetyl, propionyl or butyryl; and when it is (1-4C)alkoxy-(1-4C)alkyl is, for example, methoxymethyl, ethoxymethyl, 1-methoxyethyl, 1-ethoxyethyl, 2-methoxyethyl, 2-ethoxyethyl, 3-methoxypropyl or 3-ethoxypropyl.

When R² and R³ together form a group of the formula -A²-X²-A³- which, together with the carbon atom to which A² and A³ are attached, defines a ring having 5 to 7 ring atoms then a suitable value for A² or A³, which may be the same or different, when each is (1-3C)alkylene is, for example, methylene, ethylene or trimethylene. Suitable values for the substituents which may be present on said 5- to 7-membered ring include for example:-
- for (1-4C)alkyl:: methyl, ethyl, propyl, isopropyl, butyl and isobutyl;
- for (1-4C)alkoxy:: methoxy, ethoxy, propoxy, isopropoxy and butoxy.

A suitable pharmaceutically-acceptable salt of a novel compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically-acceptable salt of a novel compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

Particular novel compounds of the invention are, for example, cyclic ether derivatives of the formula I wherein:-
(a) Ar¹ is phenyl or naphthyl which may optionally bear one, two or three substituents selected from any of those substituents on Ar¹ defined hereinbefore other than oxo and thioxo;
(b) Ar¹ is phenyl or naphth-2-yl which may optionally bear one or two substituents selected from fluoro, chloro, methyl, ethyl, isopropyl, tert-butyl, methoxy, trifluoromethyl, 2-cyanoprop-2-yl, phenyl and benzoyl, and wherein said phenyl or benzoyl substituents may optionally bear a substituent selected from chloro, methyl and methoxy; and A¹, X¹, Ar², R¹, R² and R³ have any of the meanings defined hereinbefore;
(c) Ar¹ is a 9- or 10-membered benzo-fused heterocyclic moiety containing one or two nitrogen heteroatoms and optionally containing a futher heteroatom selected from oxygen and sulphur, which heterocyclic moiety may optionally bear one or two oxo or thioxo substituents and up to three further substituents selected from any of those substituents on Ar¹ defined hereinbefore other than oxo or thioxo; and A¹, X¹, Ar², R¹, R² and R³ have any of the meanings defined hereinbefore;
(d) Ar¹ is indolyl, indolinyl, benzimidazolyl, 2,3-dihydrobenzimidazolyl, benzoxazolyl, 2,3-dihydrobenzoxazolyl, benzothiazolyl, 2,3-dihydrobenzothiazolyl, quinolyl, 1,2-dihydroquinolyl, isoquinolyl, 1,2-dihydroisoquinolyl, quinoxalinyl, 2,3-dihydro-4H-1,4-benzoxazinyl or 2,3-dihydro-4H-1,4-benzothiazinyl, which may optionally bear one or two oxo or thioxo substituents and up to three further substituents selected from any of those substituents on Ar¹ defined hereinbefore other than oxo or thioxo; and A¹, X¹, Ar², R¹, R² and R³ have any of the meanings defined hereinbefore;
(e) Ar¹ is 2-indolyl, 3-indolyl, 5-indolyl, 6-indolyl, 2-benzimidazolyl, 5-benzimidazolyl, 6-benzimidazolyl, 2-benzoxazolyl, 5-benzoxazolyl, 6-benzoxazolyl, 2-benzothiazolyl, 5-benzothiazolyl, 6-benzothiazolyl, 2-quinolyl, 3-quinolyl, 6-quinolyl, 7-quinolyl, 3-isoquinolyl, 6-isoquinolyl, 7-isoquinolyl, 2-quinoxalinyl, 6-quinoxalinyl, 4H-1,4-benzoxazin-6-yl or 4H-1,4-benzothiazin-6-yl, which may optionally bear one or two substituents selected from any of those substituents on Ar¹ defined hereinbefore other than oxo or thioxo; and A¹, X¹, Ar², R¹, R² and R³ have any of the meanings defined hereinbefore;
(f) Ar¹ is 2-oxoindolinyl, 2,3-dioxoindolinyl, 2-oxo-2,3-dihydrobenzimidazolyl, 2-oxo-2,3-dihydrobenzoxazolyl, 2-oxo-2,3-dihydrobenzothiazolyl, 2-oxo-1,2-dihydroquinolinyl, 3-oxo-2,3-dihydro-4H-1,4-benzoxazinyl or 3-oxo-2,3-dihydro-4H-1,4-benzothiazinyl, or the corresponding thioxo derivatives thereof, which may optionally bear up to three substituents selected from any of those substituents on Ar¹ defined hereinbefore other than oxo or thioxo; and A¹, X¹, Ar², R¹, R² and R³ have any of the meanings defined hereinbefore;
(g) Ar¹ is 2-oxoindolin-5-yl, 2,3-dioxoindolin-5-yl, 2-oxo-2,3-dihydrobenzimidazol-5-yl, 2-oxo-2,3-dihydrobenzoxazol-5-yl, 2-oxo-2,3-dihydrobenzothiazol-5-yl, 2-oxo-1,2-dihydroquinolin-3-yl, 2-oxo-1,2-dihydroquinolin-6-yl, 2-oxo-1,2-dihydroquinolin-7-yl, 3-oxo-2,3-dihydro-4H-1,4-benzoxazin-7-yl or 3-oxo-2,3-dihydro-4H-1,4-benzothiazol-7-yl, which may optionally bear up to three substituents selected from any of those substituents on Ar¹ defined hereinbefore other than oxo or thioxo; and A¹, X¹, Ar², R¹, R² and R³ have any of the meanings defined hereinbefore;
(h) A¹ is a direct link to X¹, and X¹ is oxy, thio, sulphinyl or sulphonyl; and Ar¹, Ar², R¹, R² and R³ have any of the meanings defined hereinbefore;
(i) A¹ is methylene and X¹ is oxy, thio, sulphinyl or sulphonyl; and Ar¹, Ar², R¹, R² and R³ have any of the meanings defined hereinbefore;
(j) Ar² is 1,3-phenylene or 1,4-phenylene which may optionally bear one or two substituents selected from fluoro, chloro, hydroxy, amino, nitro, ureido, methyl, methoxy, methylamino, dimethylamino, trifluoromethyl and acetamido; and Ar¹, A¹, X¹, R¹, R² and R³ have any of the meanings defined hereinbefore;
(k) Ar² is 3,5-pyridylene; and Ar¹, A¹, X¹, R¹, R² and R³ have any of the meanings defined hereinbefore;
(l) R¹ is methylthio, ethylthio, propylthio, methylsulphinyl, ethylsulphinyl, propylsulphinyl, methylsulphonyl, ethylsulphonyl, propylsulphonyl, methylamino, ethylamino, dimethylamino or diethylamino; and Ar¹, A¹, X¹, Ar², R² and R³ have any of the meanings defined hereinbefore;
(m) R¹ is cyano, carbamoyl, methoxycarbonyl, ethoxycarbonyl, propoxycarbonyl, N-methylcarbamoyl, N-ethylcarbamoyl, N,N-dimethylcarbamoyl, N,N-diethylcarbamoyl, hydroxymethyl, 2-hydroxyethyl, 3-hydroxypropyl, methoxymethyl, ethoxymethyl, 2-methoxyethyl, 2-ethoxyethyl, 3-methoxypropyl or 3-ethoxypropyl; and Ar¹, A¹, X¹, Ar², R² and R³ have any of the meanings defined hereinbefore; or
(n) R¹ is hydrogen, formyl, cyano, methyl, ethyl, propyl, methoxycarbonyl, ethoxycarbonyl, propoxycarbonyl, hydroxymethyl, 1-hydroxyethyl, 2-hydroxyethyl, 2,2,2-trifluoroethyl, acetyl, propionyl, methoxymethyl, ethoxymethyl, 2-methoxyethyl or 2-ethoxyethyl; and Ar¹, A¹, X¹, Ar², R² and R³ have any of the meanings defined hereinbefore;
(o) R² and R³ together form a group of the formula -A²-X²-A³- which, together with the carbon atom to which A² and A³ are attached, defines a ring having 5 to 7 ring atoms, wherein A² and A³, which may be the same or different, each is methylene, ethylene or trimethylene and X² is oxy, and which ring may bear one or two substituents selected from hydroxy, methyl, ethyl, propyl, methoxy and ethoxy; and Ar¹, A¹, X¹, Ar² and R¹ have any of the meanings defined hereinbefore.
or a pharmaceutically-acceptable salt thereof.

A preferred compound of the invention comprises a cyclic ether derivative of the formula I
wherein Ar¹ is phenyl or naphth-2-yl which may optionally bear one or two substituents selected from fluoro, chloro, methyl, ethyl, isopropyl, tert-butyl, methoxy, trifluoromethyl, 2-cyanoprop-2-yl, phenyl and benzoyl and wherein said phenyl or benzoyl substituents may optionally bear a substituent selected from chloro, methyl and methoxy;
A¹ is a direct link to X¹, or is methylene;
X¹ is oxy, thio, sulphinyl or sulphonyl;
Ar² is 1,3-phenylene or 1,4-phenylene which may optionally bear one or two substituents selected from fluoro, chloro, hydroxy, amino, nitro, ureido, methoxy, dimethylamino, trifluoromethyl and acetamido; or Ar² is 3,5-pyridylene;
R¹ is methylthio, ethylthio, propylthio, methylsulphinyl, ethylsulphinyl, propylsulphinyl, methylsulphonyl, ethylsulphonyl, propylsulphonyl, methylamino, ethylamino, dimethylamino or diethylamino, or R¹ is cyano, carbamoyl, methoxycarbonyl, ethoxycarbonyl, propoxycarbonyl, N-methylcarbamoyl, N-ethylcarbamoyl, N,N-dimethylcarbamoyl, N,N-diethylcarbamoyl, hydroxymethyl, 2-hydroxyethyl, 3-hydroxypropyl, methoxymethyl, ethoxymethyl, 2-methoxyethyl, 2-ethoxyethyl, 3-methoxypropyl or 3-ethoxypropyl; and
R² and R³ together form a group of the formula -A²-X²-A³- which, together with the carbon atom to which A² and A³ are attached, defines a ring having 5 or 6 ring atoms, wherein A² is ethylene, A³ is methylene or ethylene, and X² is oxy, and which ring may bear one or two substituents selected from methyl and ethyl;
or a pharmaceutically-acceptable salt thereof.

A further preferred compound of the invention comprises a cyclic ether derivative of the formula I
wherein Ar¹ is 2-oxoindolin-5-yl, 2,3-dioxoindolin-5-yl, 2-oxo-2,3-dihydrobenzimidazol-5-yl, 2-oxo-2,3-dihydrobenoxazol-5-yl, 2-oxo-2,3-dihydrobenzothiazol-5-yl, 2-oxo-1,2-dihydroquinolin-3-yl, 2-oxo-1,2-dihydroquinolin-6-yl, 2-oxo-1,2-dihydroquinolin-7-yl, 3-oxo-2,3-dihydro-4H-1,4-benzoxazin-7-yl or 3-oxo-2,3-dihydro-4H-1,4-benzothiazol-7-yl, which may optionally bear one, two or three substituents selected from fluoro, chloro, methyl, ethyl, 2-fluoroethyl, phenyl and benzyl;
A¹ is a direct link to X¹, or is methylene;
X¹ is oxy, thio, sulphinyl or sulphonyl;
Ar² is 1,3-phenylene or 1,4-phenylene which may optionally bear one or two substituents selected from fluoro, chloro, hydroxy, amino, nitro, ureido, methoxy, dimethylamino, trifluoromethyl and acetamido; or Ar² is 3,5-pyridylene;
R¹ is methylthio, ethylthio, propylthio, methylsulphinyl, ethylsulphinyl, propylsulphinyl, methylsulphonyl, ethylsulphonyl, propylsulphonyl, methylamino, ethylamino, dimethylamino or diethylamino, or R¹ is cyano, carbamoyl, methoxycarbonyl, ethoxycarbonyl, propoxycarbonyl, N-methylcarbamoyl, N-ethylcarbamoyl, N,N-dimethylcarbamoyl, N,N-diethylcarbamoyl, hydroxymethyl, 2-hydroxyethyl, 3-hydroxypropyl, methoxymethyl, ethoxymethyl, 2-methoxyethyl, 2-ethoxyethyl, 3-methoxypropyl or 3-ethoxypropyl; and
R² and R³ together form a group of the formula -A²-X²-A³- which, together with the carbon atom to which A² and A³ are attached, defines a ring having 5 or 6 ring atoms, wherein A² is ethylene, A³ is methylene or ethylene, and X² is oxy, and which ring may bear one or two substituents selected from methyl and ethyl;
or a pharmaceutically-acceptable salt thereof.

A further preferred compound of the invention comprises a cyclic ether derivative of the formula I wherein Ar¹ is naphth-2-yl which may optionally bear one or two substituents selected from fluoro, chloro, methyl, methoxy and trifluoromethyl, or Ar¹ is 2-oxo-1,2-dihydroquinolin-6-yl which may optionally bear one or two substituents selected from fluoro, chloro, methyl and ethyl;
A¹ is a direct link to X¹, or is methylene;
X¹ is oxy, thio, sulphinyl or sulphonyl;
Ar² is 1,3-phenylene which may optionally bear a substituent selected from fluoro, chloro and trifluoromethyl;
R¹ is methylthio, ethylthio, propylthio, isopropylthio, tert-butylthio, methylsulphinyl, ethylsulphinyl, propylsulphinyl, hydrogen, formyl, cyano, methoxycarbonyl, ethoxycarbonyl, propoxycarbonyl, hydroxymethyl, 1-hydroxyethyl, 2-hydroxyethyl, 2,2,2-trifluoroethylthio, acetyl, propionyl, methoxymethyl, ethoxymethyl or 2-methoxyethyl; and
R² and R³ together form a group of the formula -A²-X²-A³- which, together with the carbon atom to which each of A² and A³ is attached, defines a ring having 5 or 6 ring atoms, wherein A² is ethylene, A³ is methylene or ethylene, and X² is oxy, and which ring may bear one or two substituents selected from methyl and ethyl; or a pharmaceutically-acceptable salt thereof.

A further preferred compound of the invention comprises a cyclic ether derivative of the formula I
wherein Ar¹ is phenyl which may optionally bear a substituent selected from fluoro, chloro, methyl, tert-butyl, phenyl and benzoyl, and wherein said phenyl or benzoyl substituent may optionally bear a chloro substituent, or Ar¹ is naphth-2-yl which may optionally bear a substituent selected from fluoro, chloro and methyl;
A¹ is a direct link to X¹, or is methylene;
X¹ is oxy, thio, sulphinyl or sulphonyl;
Ar² is 1,3-phenylene which may optionally bear one or two substituents selected from fluoro and trifluoromethyl;
R¹ is methylthio, ethylthio, methylsulphinyl, ethylsulphinyl, methylsulphonyl or ethylsulphonyl; and
R² and R³ together form a group of the formula -A²-X²-A³- which, together with the carbon atom to which A² and A³ is attached, defines a ring having 5 or 6 ring atoms, wherein A² is ethylene, A³ is methylene or ethylene and X² is oxy, and which ring may bear a substituent selected from methyl and ethyl;
or a pharmaceutically-acceptable salt thereof.

A further preferred compound of the invention comprises a cyclic ether derivative of the formula I
wherein Ar¹ is phenyl which may optionally bear a substituent selected from fluoro, chloro, methyl, tert-butyl, phenyl and benzoyl, and wherein said phenyl or benzoyl substituent may optionally bear a chloro substituent, or Ar¹ is naphth-2-yl which may optionally bear a substituent selected from fluoro, chloro and methyl;
A¹ is a direct link to X¹, or is methylene;
X¹ is oxy, thio, sulphinyl or sulphonyl;
Ar² is 1,3-phenylene which may optionally bear one or two substituents selected from fluoro and trifluoromethyl;
R¹ is cyano, methoxycarbonyl, ethoxycarbonyl, hydroxymethyl, 2-hydroxyethyl, methoxymethyl, ethoxymethyl, 2-methoxyethyl or 2-ethoxyethyl; and
R² and R³ together form a group of the formula -A²-X²-A³- which, together with the carbon atom to which A² and A³ is attached, defines a ring having 5 or 6 ring atoms, wherein A² is ethylene, A³ is methylene or ethylene and X² is oxy, and which ring may bear a substituent selected from methyl and ethyl;
or a pharmaceutically-acceptable salt thereof.

A further preferred compound of the invention comprises a cyclic ether derivative of the formula I
wherein Ar¹ is 1,3-dimethyl-2-oxo-2,3-dihydrobenzimidazol-5-yl, 3-methyl-2-oxo-2,3-dihydrobenzothiazol-6-yl, 1-methyl-2-oxo-1,2-dihydroquinolin-6-yl, 1-ethyl-2-oxo-1,2-dihydroquinolin-6-yl, 4-methyl-3-oxo-2,3-dihydro-4H-1,4-benzoxazin-7-yl or 2,2,4-trimethyl-3-oxo-2,3-dihydro-4H-1,4-benzoxazin-7-yl;
A¹ is a direct link to X¹, or is methylene;
X¹ is oxy, thio, sulphinyl or sulphonyl;
Ar² is 1,3-phenylene which may optionally bear one or two substituents selected from fluoro and trifluoromethyl;
R¹ is methylthio, ethylthio, methylsulphinyl, ethylsulphinyl, methylsulphonyl or ethylsulphonyl; and
R² and R³ together form a group of the formula -A²-X²-A³- which, together with the carbon atom to which A² and A³ is attached, defines a ring atom having 5 or 6 ring atoms, wherein A² is ethylene, A³ is methylene or ethylene and X² is oxy, and which ring may bear a substituent selected from methyl and ethyl;
or a pharmaceutically-acceptable salt thereof.

A further preferred compound of the invention comprises a cyclic ether derivative of the formula I
wherein Ar¹ is 1,3-dimethyl-2-oxo-2,3-dihydrobenzimidazol-5-yl, 3-methyl-2-oxo-2,3-dihydrobenzothiazol-6-yl, 1-methyl-2-oxo-1,2-dihydroquinolin-6-yl, 1-ethyl-2-oxo-1,2-dihydroquinolin-6-yl, 4-methyl-3-oxo-2,3-dihydro-4H-1,4-benzoxazin-7-yl or 2,2,4-trimethyl-3-oxo-2,3-dihydro-4H-1,4-benzoxazin-7-yl;
A¹ is a direct link to X¹, or is methylene;
X¹ is oxy, thio, sulphinyl or sulphonyl;
Ar² is 1,3-phenylene which may optionally bear one or two substituents selected from fluoro and trifluoromethyl;
R¹ is cyano, methoxycarbonyl, ethoxycarbonyl, hydroxymethyl or methoxymethyl; and
R² and R³ together form a group of the formula -A²-X²-A³- which, together with the carbon atom to which A² and A³ is attached, defines a ring having 5 or 6 ring atoms, wherein A² is ethylene, A³ is methylene or ethylene and X² is oxy, and which ring may bear a substituent selected from methyl and ethyl;
or a pharmaceutically-acceptable salt thereof.

A further preferred compound of the invention comprises a cyclic ether derivative of the formula I
wherein Ar¹ is naphth-2-yl which may optionally bear a fluoro substituent, or Ar¹ is 1-methyl-2-oxo-1,2-dihydroquinolin-6-yl;
A¹ is methylene and X¹ is oxy;
Ar² is 1,3-phenylene or 5-fluoro-1,3-phenylene;
R¹ is methylthio, ethylthio, isopropylthio, tert-butylthio, methylsulphinyl, hydrogen, formyl, cyano, methyl, ethyl, ethoxycarbonyl, hydroxymethyl, 1-hydroxyethyl, 2,2,2-trifluoroethylthio, acetyl or methoxymethyl; and
R² and R³ together form a group of the formula -A²-X²-A³- which, together with the carbon atom to which each of A² and A³ is attached, defines a ring having 6 ring atoms, wherein A² is ethylene, A³ is ethylene and X² is oxy, and which ring may bear a methyl substituent alpha to X²;
or a pharmaceutically-acceptable salt thereof.

Specific especially preferred compounds of the invention include the following cyclic ether derivatives of the formula I, or pharmaceutically-acceptable salts thereof:-
4-methylthio-4-[3-(naphth-2-ylmethoxy)phenyl]tetrahydropyran and 4-ethoxycarbonyl-4-[3-(1-methyl-2-oxo-1,2-dihydroquinolin-6-ylmethoxy)phenyl]tetrahydropyran.

Further specific especially preferred compounds of the invention include the following cyclic ether derivatives of the formula I, or pharmaceutically-acceptable salts thereof:-
4-acetyl-4-[3-(1-methyl-2-oxo-1,2-dihydroquinolin-6-ylmethoxy)phenyl]tetrahydropyran,
4-[5-fluoro-3-(1-methyl-2-oxo-1,2-dihydroquinolin-6-ylmethoxy)phenyl]-3,4,5,6-tetrahydro-2H-pyran,
4-ethyl-4-[3-(1-methyl-2-oxo-1,2-dihydroquinolin-6-ylmethoxy)phenyl]tetrahydropyran,
(2RS,4RS)-4-ethyl-4-[5-fluoro-3-(1-methyl-2-oxo-1,2-dihydroquinolin-6-ylmethoxy)phenyl]-2-methyltetrahydropyran and
4-[5-fluoro-3-(1-methyl-2-oxo-1,2-dihydroquinolin-6-ylmethoxy)phenyl]-4-methylthiotetrahydropyran.

A compound of the invention comprising a cyclic ether derivative of the formula I, or a pharmaceutically-acceptable salt thereof, may be prepared by any process known to be applicable to the preparation of structurally-related compounds. Such procedures are provided as a further feature of the invention and are illustrated by the following representative examples in which, unless otherwise stated, Ar¹, A¹, X¹, Ar², R¹, R² and R³ have any of the meanings defined hereinbefore.
(a) The coupling, preferably in the presence of a suitable base, of a compound of the formula Ar¹-A¹-X¹-H with a compound of the formula II wherein Z is a displaceable group; provided that, when there is an amino, alkylamino or hydroxy group in Ar¹, Ar², R¹, R² or R³, any amino, alkylamino or hydroxy group may be protected by a conventional protecting group or alternatively any such group need not be protected, whereafter any undesired protecting group in Ar¹, Ar², R¹, R² or R³ is removed by conventional means.
   A suitable displaceable group Z is, for example, a halogeno or sulphonyloxy group, for example a chloro, bromo, iodo, methane-sulphonyloxy or toluene-p-sulphonyloxy group.
   A suitable base for the coupling reaction is, for example, an alkali or alkaline earth metal carbonate, (1-4C)alkoxide, hydroxide or hydride, for example sodium carbonate, potassium carbonate, sodium ethoxide, sodium butoxide, sodium hydroxide, potassium hydroxide, sodium hydride or potassium hydride. The coupling reaction is conveniently performed in a suitable inert solvent or diluent, for example N,N-dimethylformamide, N,N-dimethylacetamide, N-methylpyrrolidin-2-one, dimethylsulphoxide, acetone, 1,2-dimethoxyethane or tetrahydrofuran, and at a temperature in the range, for example, 10 to 150°C, conveniently at or near 100°C.
   Conveniently the reaction may be performed in the presence of a suitable catalyst, for example a metallic catalyst, for example palladium(O) or copper(I) such as tetrakis(triphenylphosphine)palladium, cuprous chloride or cuprous bromide.
   A suitable protecting group for an amino or alkylamino group is, for example, an acyl group for example a (2-4C)alkanoyl group (especially acetyl), a (1-4C)alkoxycarbonyl group (especially methoxycarbonyl, ethoxycarbonyl or tert-butoxycarbonyl), an arylmethoxycarbonyl group (especially benzyloxycarbonyl) or an aroyl group (especially benzoyl). The deprotection conditions for the above protecting groups necessarily vary with the choice of protecting group. Thus, for example, an acyl group such as an alkanoyl or alkoxycarbonyl or an aroyl group may be removed for example, by hydrolysis with a suitable base such as an alkali metal hydroxide, for example lithium or sodium hydroxide. Alternatively an acyl group such as a tert-butoxycarbonyl group may be removed, for example, by treatment with a suitable acid such as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-charcoal.
   A suitable protecting group for a hydroxy group is, for example, an acyl group, for example a (2-4C)alkanoyl group (especially acetyl), an aroyl group (especially benzoyl) or an arylmethyl group (especially benzyl). The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-charcoal.
   The starting materials of the formula Ar¹-A¹-X¹-H and of the formula II may be obtained by standard procedures of organic chemistry.
(b) The coupling, preferably in the presence of a suitable base as defined hereinbefore, of a compound of the formula III with a compound of the formula Ar¹-A¹-Z wherein Z is a displaceable group as defined hereinbefore; provided that, when there is an amino, alkylamino or hydroxy group in Ar¹, Ar², R¹, R² or R³, any amino, alkylamino or hydroxy group may be protected by a conventional protecting group as defined hereinbefore or alternatively any such group need not be protected, whereafter any undesired protecting group in Ar¹, Ar², R¹, R² or R³ is removed by conventional means.
   The coupling reaction is conveniently performed in a suitable inert solvent as defined hereinbefore and at a temperature in the range, for example, 10 to 150°C, conveniently at or near 100°C. The reaction may conveniently be performed in the presence of a suitable catalyst as defined hereinbefore.
   The starting materials of the formula Ar¹-A¹-Z and of the formula III may be obtained by standard procedures of organic chemistry. The preparation of such starting materials is described within the accompanying non-limiting Examples which are provided for the purpose of illustration only. Other necessary starting materials are obtainable by analogous procedures to those described or by modifications thereto which are within the ordinary skill of an organic chemist.
(c) For the production of those compounds of the formula I wherein R¹ is (1-4C)alkylthio, the alkylation, preferably in the presence of a suitable base as defined hereinbefore, of a compound of the formula IV with a compound of the formula R⁴-Z, wherein Z has the meaning defined hereinbefore and R⁴ is a (1-4C)alkyl group; provided that, when there is an amino, imino, alkylamino or hydroxy group in Ar¹, Ar², X¹, R² or R³ any amino, imino, alkylamino or hydroxy group may be protected by a conventional protecting group or alternatively any such group need not be protected, whereafter any undesired protecting group in Ar¹, Ar², X¹, R² or R³ is removed by conventional means.
   A suitable protecting group for an imino group is, for example, any of the protecting groups defined hereinbefore for an amino or alkylamino group.
   The starting material of the formula IV may be obtained by standard procedures of organic chemistry. The preparation of such starting materials is described within the accompanying non-limiting Examples which are provided for the purpose of illustration only.
(d) For the production of those compounds of the formula I wherein X¹ is a sulphinyl or sulphonyl group, wherein R¹ is a (1-4C)alkylsulphinyl or (1-4C)alkylsulphonyl group, or wherein R² and R³ together form a group of the formula -A²-X²-A³- and X² is a sulphinyl or sulphonyl group, the oxidation of a compound of the formula I wherein X¹ is a thio group, wherein R¹ is a (1-4C)alkylthio group, or wherein R² and R³ together form a group of the formula -A²-X²-A³- and X² is a thio group.
   A suitable oxidising agent is, for example, any agent known in the art for the oxidation of thio to sulphinyl and/or sulphonyl, for example, hydrogen peroxide, a peracid (such as 3-chloroperoxybenzoic or peroxyacetic acid), an alkali metal peroxysulphate (such as potassium peroxymonosulphate), chromium trioxide or gaseous oxygen in the presence of platinum. The oxidation is generally carried out under as mild conditions as possible and with the required stoichiometric amount of oxidising agent in order to reduce the risk of over oxidation and damage to other functional groups. In general the reaction is carried out in a suitable solvent or diluent such as methylene chloride, chloroform, acetone, tetrahydrofuran or tert-butyl methyl ether and at a temperature, for example, at or near ambient temperature, that is in the range 15 to 35°C. When a compound carrying a sulphinyl group is required a milder oxidising agent may also be used, for example sodium or potassium metaperiodate, conveniently in a polar solvent such as acetic acid or ethanol. It will be appreciated that when a compound of the formula I containing a sulphonyl group is required, it may be obtained by oxidation of the corresponding sulphinyl compound as well as of the corresponding thio compound.
(e) For the production of those compounds of the formula I wherein Ar² bears an alkanoylamino substituent, the acylation of a compound of the formula I wherein Ar² bears an amino substituent.
   A suitable acylating agent is, for example, any agent known in the art for the acylation of amino to acylamino, for example an acyl halide, for example a (2-6C)alkanoyl chloride or bromide, in the presence of a suitable base, an alkanoic acid anhydride, for example a (2-6C)alkanoic acid anhydride, or an alkanoic acid mixed anhdride, for example the mixed anhydride formed by the reaction of an alkanoic acid and a (1-4C)alkoxycarbonyl halide, for example a (1-4C)alkoxycarbonyl chloride, in the presence of a suitable base. In general the reaction is carried out in a suitable solvent or diluent such as methylene chloride, acetone, tetrahydrofuran or tert-butyl methyl ether and at a temperature, for example, at or near ambient temperature, that is in the range 15 to 35°C. A suitable base when it is required is, for example, pyridine, 4-dimethylaminopyridine, triethylamine, ethyldiisopropylamine, N-methylmorpholine, an alkali metal carbonate, for example potassium carbonate, or an alkali metal carboxylate, for example sodium acetate.
(f) For the production of those compounds of the formula I wherein Ar¹ bears an alkyl or substituted alkyl substituent on an available nitrogen atom, wherein Ar² bears an alkoxy substituent or wherein R¹ is an alkoxyalkyl group, the alkylation of a compound of the formula I wherein Ar¹ bears a hydrogen atom on said available nitrogen atom, wherein Ar² bears a hydroxy substituent or wherein R¹ is a hydroxyalkyl group.

A suitable alkylating agent is, for example, any agent known in the art for the alkylation of an available nitrogen atom, or of hydroxy to alkoxy, for example an alkyl or substituted alkyl halide, for example a (1-6C)alkyl chloride, bromide or iodide or a substituted (1-4C)alkyl chloride, bromide or iodide, in the presence of a suitable base. A suitable base for the alkylation reaction is, for example, an alkali or alkaline earth metal carbonate, sodium hydroxide, potassium hydroxide, sodium hydride or potassium hydride. The alkylation reaction is preferably performed in a suitable inert solvent or diluent, for example N,N-dimethylformamide, dimethylsulphoxide, acetone, 1,2-dimethoxyethane or tetrahydrofuran, and at a temperature in the range, for example, 10 to 150°C, conveniently at or near ambient temperature.

When a pharmaceutically-acceptable salt of a novel compound of the formula I is required, it may be obtained, for example, by reaction of said compound with a suitable acid or base using a conventional procedure. When an optically active form of a compound of the formula I is required, it may be obtained by carrying out one of the aforesaid procedures using an optically active starting material, or by resolution of a racemic form of said compound using a conventional procedure.

Many of the intermediates defined herein are novel, for example those of the formula IV and this is provided as a further feature of the invention.

As stated previously, the novel compounds of the formula I are inhibitors of the enzyme 5-LO. The effects of this inhibition may be demonstrated using one or more of the standard procedures set out below:-
a) An in vitro spectrophotometric enzyme assay system, which assesses the inhibitory properties of a test compound in a cell free system using 5-LO isolated from guinea pig neutrophils and as described by D. Aharony and R.L. Stein (J. Biol. Chem., 1986, 261(25), 11512-11519). This test provides a measure of the intrinsic inhibitory properties of a test compound against soluble 5-LO in an extracellular environment.
b) An in vitro assay system involving incubating a test compound with heparinised human blood, prior to challenge with the calcium ionophore A23187 and then indirectly measuring the inhibitory effects on 5-LO by assaying the amount of LTB₄ using specific radioimmunoassays described by Carey and Forder (F. Carey and R.A. Forder, Prostaglandins, Leukotrienes Med., 1986, 22, 57; Prostaglandins, 1984, 28, 666; Brit. J. Pharmacol. 1985, 84, 34P) which involve the use of a protein-LTB₄ conjugate produced using the procedure of Young et alia (Prostaglandins, 1983, 26(4), 605-613). The effects of a test compound on the enzyme cyclooxygenase (which is involved in the alternative metabolic pathway for arachidonic acid and gives rise to prostaglandins, thromboxanes and related metabolites) may be measured at the same time using the specific radioimmunoassay for thromboxane B₂(TxB₂) described by Carey and Forder (see above). This test provides an indication of the effects of a test compound against 5-LO and also cyclooxygenase in the presence of blood cells and proteins. It permits the selectivity of the inhibitory effect on 5-LO or cyclooxygenase to be assessed.
c) An ex vivo assay system, which is a variation of test b) above, involving administration of a test compound (usually orally as the suspension produced when a solution of the test compound in dimethylsulphoxide is added to carboxymethylcellulose), blood collection, heparinisation, challenge with A23187 and radioimmunoassay of LTB₄ and TxB₂. This test provides an indication of the bioavailability of a test compound as an inhibitor of 5-LO or cyclooxygenase.
d) An in vitro assay system involving the measurement of the inhibitory properties of a test compound against the liberation of LTC₄ and PGE₂ induced by zymosan on mouse resident peritoneal macrophages, using the procedure of Humes (J.L. Humes et alia, Biochem. Pharmacol., 1983, 32, 2319-2322) and conventional radioimmunoassay systems to measure LTC₄ and PGE₂. This test provides an indication of inhibitory effects against 5-LO and cyclooxygenase in a non-proteinaceous system.
e) An in vivo system involving the measurement of the effects of a test compound in inhibiting the inflammatory response to arachidonic acid in the rabbit skin model developed by D. Aked et alia (Brit. J. Pharmacol., 1986, 89, 431-438). This test provides an in vivo model for 5-LO inhibitors administered topically or orally.
f) An in vivo system involving measuring the effects of a test compound administered orally or intravenously on a leukotriene dependent bronchoconstriction induced by an antigen challenge in guinea pigs pre-dosed with an antihistamine (mepyramine), a β-adrenergic blocking agent (propranolol) and a cyclooxygenase inhibitor (indomethacin), using the procedure of W.H. Anderson et alia (British J. Pharmacology, 1983, 78(1), 67-574). This test provides a further in vivo test for detecting 5-LO inhibitors.
g) An in vivo system involving measuring the effects of a test compound administered orally against the liberation of LTB₄ induced by zymosan within an air pouch generated within the subcutaneous tissue of the back of male rats. The rats are anaesthetised and air pouches are formed by the injection of sterile air (20ml). A further injection of air (10ml) is similarly given after 3 days. At 6 days after the initial air injection the test compound is administered (usually orally as the suspension produced when a solution of the test compound in dimethylsulphoxide is added to hydroxypropylmethylcellulose), followed by the intrapouch injection of zymosan (1ml of a 1% suspension in physiological saline). After 3 hours the rats are killed, the air pouches are lavaged with physiological saline, and the specific radioimmunoassay described above is used to assay LTB₄ in the washings. This test provides an indication of inhibitory effects against 5-LO in an inflammatory milieu.

Although the pharmacological properties of the compounds of the formula I vary with structural changes as expected, in general compounds of the formula I possess 5-LO inhibitory effects at the following concentrations or doses in one or more of the above tests a)-g):-
- Test a):: IC₅₀ in the range, for example, 0.01-30µM;
- Test b):: IC₅₀ (LTB₄) in the range, for example, 0.01-40µM IC₅₀ (TxB₂) in the range, for example, 40-200µM;
- Test c):: oral ED₅₀(LTB₄) in the range, for example, 1-100mg/kg;
- Test d):: IC₅₀ (LTC₄) in the range, for example, 0.001-1µM, IC₅₀ (PGE₂) in the range, for example, 20-1000µM;
- Test e):: inhibition of inflammation in the range, for example, 0.3-100µg intradermally;
- Test f):: ED₅₀ in the range, for example, 0.5-10mg/kg i.v.;
- Test g):: oral ED₅₀(LTB₄) in the range, for example, 0.5-50mg/kg.

No overt toxicity or other untoward effects are present in tests c), e), f) and/or g) when compounds of the formula I are administered at several multiples of their minimum inhibitory dose or concentration.

Thus, by way of example, the compound 4-methylthio-4-[3-(naphth-2-ylmethoxy)phenyl]tetrahydropyran has an IC₅₀ of 0.05µM against LTB₄ in test b), and an oral ED₅₀ of 8mg/kg versus LTB₄ in test g); and the compound 4-ethoxycarbonyl-4-[3-(1-methyl-2-oxo-1,2-dihydroquinolin-6-ylmethoxy)tetrahydropyran has an IC₅₀ of 0.05µM aganst LTB₄ in test b). In general those compounds of the formula I which are particularly preferred have an IC₅₀ of <1µM against LTB₄ in test b), and an oral ED₅₀ of <100 mg/kg against LTB₄ in tests c) and/or g).

These compounds are examples of compounds of the invention which show selective inhibitory properties for 5-LO as opposed to cyclooxygenase, which selective properties are expected to impart improved therapeutic properties, for example, a reduction in or freedom from the gastrointestinal side-effects frequently associated with cyclooxygenase inhibitors such as indomethacin.

According to a further feature of the invention there is provided a pharmaceutical composition which comprises a cyclic ether derivative of the formula I, or a pharmaceutically-acceptable salt thereof, in association with a pharmaceutically-acceptable diluent or carrier.

The composition may be in a form suitable for oral use, for example a tablet, capsule, aqueous or oily solution, suspension or emulsion; for topical use, for example a cream, ointment, gel or aqueous or oily solution or suspension; for nasal use, for example a snuff, nasal spray or nasal drops; for vaginal or rectal use, for example a suppository; for administration by inhalation, for example as a finely divided powder or a liquid aerosol; for sub-lingual or buccal use, for example a tablet or capsule; or for parenteral use (including intravenous, subcutaneous, intramuscular, intravascular or infusion), for example a sterile aqueous or oily solution or suspension. In general the above compositions may be prepared in a conventional manner using conventional excipients.

The amount of active ingredient (that is a cyclic ether derivative of the formula I, or a pharmaceutically-acceptable salt thereof) that is combined with one or more excipients to produce a single dosage form will necessarily vary depending upon the host treated and the particular route of administration. For example, a formulation intended for oral administration to humans will generally contain, for example, from 0.5 mg to 2g of active agent compounded with an appropriate and convenient amount of excipients which may vary from about 5 to about 98 percent by weight of the total composition. Dosage unit forms will generally contain about 1 mg to about 500 mg of an active ingredient.

According to a further feature of the invention there is provided a cyclic ether derivative of the formula I, or a pharmaceutically-acceptable salt thereof, for use in a method of treatment of the human or animal body by therapy.

The invention also includes a method of treating a disease or medical condition mediated alone or in part by one or more leukotrienes which comprises administering to a warm-blooded animal requiring such treatment an effective amount of an active ingredient as defined above. The invention also provides the use of such an active ingredient in the production of a new medicament for use in a leukotriene mediated disease or medical condition.

The size of the dose for therapeutic or prophylactic purposes of a compound of the formula I will naturally vary according to the nature and severity of the conditions, the age and sex of the animal or patient and the route of administration, according to well known principles of medicine. As mentioned above, compounds of the formula I are useful in treating those allergic and inflammatory conditions which are due alone or in part to the effects of the metabolites of arachidonic acid arising by the linear (5-LO catalysed) pathway and in particular the leukotrienes, the production of which is mediated by 5-LO. As previously mentioned, such conditions include, for example, asthmatic conditions, allergic reactions, allergic rhinitis, allergic shock, psoriasis, atopic dermatitis, cardiovascular and cerebrovascular disorders of an inflammatory nature, arthritic and inflammatory joint disease, and inflammatory bowel diseases.

In using a compound of the formula I for therapeutic or prophylactic purposes it will generally be administered so that a daily dose in the range, for example, 0.5mg to 75mg per kg body weight is received, given if required in divided doses. In general lower doses will be administered when a parenteral route is employed. Thus, for example, for intravenous administration, a dose in the range, for example, 0.5mg to 30 mg per kg body weight will generally be used. Similarly, for administration by inhalation, a dose in the range, for example, 0.5 mg to 25 mg per kg body weight will be used.

Although the compounds of the formula I are primarily of value as therapeutic agents for use in warm-blooded animals (including man), they are also useful whenever it is required to inhibit the enzyme 5-LO. Thus, they are useful as pharmacological standards for use in the development of new biological tests and in the search for new pharmacological agents.

By virtue of their effects on leukotriene production, the compounds of the formula I have certain cytoprotective effects, for example they are useful in reducing or suppressing certain of the adverse gastrointestinal effects of the cyclooxygenase inhibitory non-steroidal anti-inflammatory agents (NSAIA), such as indomethacin, acetylsalicylic acid, ibuprofen, sulindac, tolmetin and piroxicam. Furthermore, co-administration of a 5-LO inhibitor of the formula I with a NSAIA can result in a reduction in the quantity of the latter agent needed to produce a therapeutic effect, thereby reducing the likelihood of adverse side-effects. According to a further feature of the invention there is provided a pharmaceutical composition which comprises a cyclic ether derivative of the formula I, or a pharmaceutically-acceptable salt thereof as defined hereinbefore, in conjunction or admixture with a cyclooxygenase inhibitory non-steroidal anti-inflammatory agent (such as mentioned above), and a pharmaceutically-acceptable diluent or carrier.

The cytoprotective effects of the compounds of the formula I may be demonstrated, for example in a standard laboratory model which assesses protection against indomethacin-induced or ethanol-induced ulceration in the gastrointestinal tract of rats.

The compositions of the invention may in addition contain one or more therapeutic or prophylactic agents known to be of value for the disease under treatment. Thus, for example a known platelet aggregation inhibitor, hypolipidemic agent, anti-hypertensive agent, beta-adrenergic blocker or a vasodilator may usefully also be present in a pharmaceutical composition of the invention for use in treating a heart or vascular disease or condition. Similarly, by way of example, an anti-histamine, steroid (such as beclomethasone dipropionate), sodium cromoglycate, phosphodiesterase inhibitor or a beta-adrenergic stimulant may usefully also be present in a pharmaceutical composition of the invention for use in treating a pulmonary disease or condition.

The invention will now be illustrated in the following non-limiting Examples in which, unless otherwise stated:-
(i) evaporations were carried out by rotary evaporation in vacuo and work-up procedures were carried out after removal of residual solids by filtration;
(ii) operations were carried out at room temperature, that is in the range 18-25° and under an atmosphere of an inert gas such as argon;
(iii) column chromatography (by the flash procedure) and medium pressure liquid chromatography (MPLC) were performed on Merck Kieselgel silica (Art. 9385) or Merck Lichroprep RP-18 (Art. 9303) reversed-phase silica obtained from E. Meck, Darmstadt, W. Germany;
(iv) yields are given for illustration only and are not necessarily the maximum attainable;
(v) the end-products of the formula I have satisfactory microanalyses and their structures were confirmed by NMR and mass spectral techniques;
(vi) intermediates were not generally fully characterised and purity was assessed by thin layer chromatographic, infra-red (IR) or NMR analysis;
(vii) melting points are uncorrected and were determined using a Mettler SP62 automatic melting point apparatus or an oil-bath apparatus; melting points for the end-products of the formula I were determined after recrystallisation from a conventional organic solvent such as ethanol, methanol, acetone, ether or hexane, alone or in admixture; and
(viii) the following abbreviations have been used:-
   - THF: tetrahydrofuran;
   - DMSO: dimethylsulphoxide;
   - DMF: N,N-dimethylformamide;
   - DMA: N,N-dimethylacetamide.

### EXAMPLE 1

Sodium hydride (50% w/w dispersion in mineral oil, 0.05 g) was added to a solution of 4-mercapto-4-[3-(naphth-2-ylmethoxy)phenyl]tetrahydropyran (0.32 g) in DMF (3 ml) and the mixture was stirred at ambient temperature for 30 minutes. Methyl iodide (0.2 ml) was added and the mixture was stirred at ambient temperature for 1 hour. The mixture was poured onto water (10 ml) and neutralised by the addition of dilute aqueous hydrochloric acid. The mixture was extracted with diethyl ether. The organic extract was washed with water, dried (MgSO₄) and evaporated. The residue was purified by column chromatography using a 9:1 v/v mixture of hexane and ethyl acetate as eluent. There was thus obtained 4-methylthio-4-[3-(naphth-2-ylmethoxy)phenyl]tetrahydropyran (0.217 g, 65%), m.p. 95-97°C.

The 4-mercapto-4-[3-(naphth-2-ylmethoxy)phenyl]tetrahydropyran used as a starting material was obtained as follows:-
A Grignard reagent was prepared by heating a mixture of 3-(naphth-2-ylmethoxy)bromobenzene (3 g), magnesium powder (0.23 g) and THF (12 ml) to 30°C for 1.5 hours. The reagent was cooled to 20°C and a solution of tetrahydropyran-4-one (0.88 ml) in THF (5 ml) was added dropwise. The mixture was heated to 30°C for 15 hours, evaporated and the residue was partitioned between ethyl acetate and water. The organic layer was separated, washed with brine, dried (MgSO₄) and evaporated. The residue was purified by column chromatography using a 7:3 v/v mixture of methylene chloride and diethyl ether as eluent. There was thus obtained 4-hydroxy-4-[3-(naphth-2-ylmethoxy)phenyl]tetrahydropyran (2.06 g, 42%), m.p. 130-131°C.

A mixture of a portion (1.67 g) of the product so obtained, Lawesson's reagent [2,4-bis-(4-methoxyphenyl)-1,3-dithia-2,4-diphosphetane-2,4-disulphide; 1.0 g] and methylene chloride (30 ml) was stirred at ambient temperature for 1.5 hours. The mixture was evaporated and the residue was partitioned between diethyl ether and water. The organic phase was washed with water and brine, dried (MgSO₄) and evaporated. The residue was purified by column chromatography using a 19:1 v/v mixture of toluene and ethyl acetate as eluent. There was thus obtained the required starting material (0.32 g, 18%), m.p. 106-109°C.

### EXAMPLE 2

A mixture of 6-bromomethyl-1-methyl-1,2-dihydroquinolin-2-one (0.1 g), 4-ethoxycarbonyl-4-(3-hydroxyphenyl)tetrahydropyran (0.098 g), potassium carbonate (0.057 g) and DMF (2 ml) was stirred at ambient temperature for 16 hours. The mixture was partitioned between diethyl ether and 0.5N aqueous hydrochloric acid. The organic layer was washed with water and brine, dried (MgSO₄) and evaporated. The residue was purified by column chromatography using initially methylene chloride and then increasingly polar mixtures of methylene chloride and acetone as eluent. There was thus obtained an oil which was triturated in pentane to give 4-ethoxycarbonyl-4-[3-(1-methyl-2-oxo-1,2-dihydroquinolin-6-ylmethoxy)phenyl]tetrahydropyran (0.099 g, 60%), m.p. 109-110°C.
NMR Spectrum (CDCl₃, δ values) 1.15 (t, 3H), 1.8-2.65 (m, 4H), 3.40-4.10 (m, 4H), 3.75 (s, 3H), 4.15 (q, 2H), 5.10 (s, 2H), 6.65-7.80 (m, 9H).

The 6-bromomethyl-1-methyl-1,2-dihydroquinolin-2-one, used as a starting material was obtained as follows:-
A mixture of 1,2-dihydro-1,6-dimethylquinolin-2-one (4.4 g; Helv. Chim. Acta., 1970, 53, 1903), N-bromosuccinimide (4.53 g), azobisisobutyronitrile (0.01 g) and carbon tetrachloride (75 ml) was heated to reflux for 3 hours. The mixture was evaporated and the residue was partitioned between ethyl acetate and water. The organic phase was washed with water, dried (MgSO₄) and evaporated. The residue was purified by column chromatography using a 2:1 v/v mixture of toluene and ethyl acetate as eluent. There was thus obtained the required starting material (4.8 g, 75%), as a solid, m.p. 107-108°C.
NMR Spectrum (CDCl₃, δ values) 3.7 (s, 3H), 4.57 (s, 2H), 6.7-7.5 (d, 1H), 7.25-7.65 (m, 4H).

The 4-ethoxycarbonyl-4-(3-hydroxyphenyl)tetrahydropyran used as a starting material was obtained as follows:-
A mixture of 3-hydroxyphenylacetic acid (10 g), ethanol (150 ml) and concentrated sulphuric acid (0.5 ml) was stirred at ambient temperature for 16 hours. The mixture was evaporated and the residue was partitioned between methylene chloride and water. The organic phase was washed with water and brine, dried (MgSO₄) and evaporated. The residue was purified by column chromatography using initially methylene chloride and then increasingly polar mixtures of methylene chloride and acetone as eluent. There was thus obtained ethyl 3-hydroxyphenylacetate (11.4 g, 96%), IR Spectrum 1690-1740 cm⁻¹.

A mixture of the product so obtained, benzyl bromide (10.82 g), potassium carbonate (9.6 g) and DMF (110 ml) was stirred at ambient temperature for 16 hours. The mixture was partitioned between diethyl ether and water. The organic phase was washed with water and brine, dried (MgSO₄) and evaporated. The residue was purified by column chromatography using methylene chloride as eluent. There was thus obtained ethyl 3-benzyloxyphenylacetate (13.8 g, 80%), IR Spectrum 1720-1740 cm⁻¹.

Sodium hydride (60% w/w dispersion in mineral oil, 0.176 g) was added to a mixture of the ester so obtained (0.54 g), 1,4,7,10,13-pentaoxacyclopentadecane (hereinafter 15-crown-5, 3 drops) and DMF (10 ml) and the mixture was stirred at ambient temperature for 25 minutes. Sodium iodide (0.3 g) and bis-2-chloroethyl ether (0.488 g) were added in turn and the mixture was stirred at ambient temperature for 16 hours. The mixture was partitioned between diethyl ether and 0.5N aqueous hydrochloric acid. The organic phase was washed with water and brine, dried (MgSO₄) and evaporated. The residue was purified by column chromatography using initially methylene chloride and then a 19:1 v/v mixture of methylene chloride and diethyl ether as eluent. There was thus obtained 4-(3-benzyloxyphenyl)-4-ethoxycarbonyltetrahydropyran (0.325 g, 48%), m.p. 70-71°C.

A mixture of a portion (0.3 g) of the product so obtained, 10% palladium-on-charcoal catalyst (0.05 g) and ethanol (8 ml) was stirred under 2 atmospheres of hydrogen for 16 hours. The mixture was filtered and evaporated. The residue was purified by column chromatography using increasingly polar mixtures of methylene chloride and acetone as eluent. There was thus obtained the required starting material (0.2 g, 91%), m.p. 100-101°C.

### EXAMPLE 3

The procedure described in Example 2 was repeated except that 2-bromomethyl-7-fluoronaphthalene was used in place of 6-bromomethyl-1-methyl-1,2-dihydroquinolin-2-one. There was thus obtained 4-ethoxycarbonyl-4-[3-(7-fluoronaphth-2-ylmethoxy)phenyl]tetrahydropyran in 76% yield, m.p. 91-92°C.
Elemental Analysis: Found C, 73.0; H, 6.2; C₂₅H₂₅FO₄ requires C, 73.5; H, 6.1%.

The 2-bromomethyl-7-fluoronaphthalene used as a starting material was obtained as follows:-
3-Fluorobenzyl chloride was reacted with acetylacetaldehyde dimethyl acetal using the procedure described for the corresponding reaction of 3-methylbenzyl chloride (Synthesis, 1974, 566). There was thus obtained 4-(3-fluorophenyl)-3-hydroxy-3-methylbutanal dimethyl acetal (b.p. 125-135°C at 0.25 mm Hg). A mixture of the material so obtained (15 g), glacial acetic acid (60 ml) and hydrobromic acid (48% w/v. 48 ml) was heated on a steam bath for 1 hour. The mixture was evaporated and the residue was purified by column chromatography using petroleum ether (b.p. 60-80°C) as eluent. There was thus obtained 7-fluoro-2-methylnaphthalene (4 g).

A mixture of 7-fluoro-2-methylnaphthalene (3 g), N-bromosuccinimide (3.3 g), 2,2'-azobisisobutyronitrile (0.2 g) and carbon tetrachloride (100 ml) was heated to reflux for 1 hour. The mixture was cooled to ambient temperature and filtered. The filtrate was evaporated and the residue was purified by column chromatography using a 19:1 v/v mixture of petroleum ether (b.p. 60-80°C) and toluene as eluent. There was thus obtained 2-bromomethyl-7-fluoronaphthalene (2.8 g), m.p. 62°C.

### EXAMPLE 4

Using the procedure described in the third paragraph of the portion of Example 2 which is concerned with the preparation of 4-ethoxycarbonyl-4-(3-hydroxyphenyl)tetrahydropyran, 3-(1-methyl-2-oxo-1,2-dihydroquinolin-6-ylmethoxy)phenylacetonitrile was reacted with bis-2-chloroethyl ether to give 4-cyano-4-[3-(1-methyl-2-oxo-1,2-dihydroquinolin-6-ylmethoxy)phenyl]tetrahydropyran in 61% yield, m.p. 149-150°C (recrystallised from a mixture of methylene chloride and diethyl ether).
IR Spectrum 2240 cm⁻¹.

The 3-(1-methyl-2-oxo-1,2-dihydroquinolin-6-ylmethoxy)phenylacetonitrile used as a starting material was obtained as follows:-
The procedure described in Example 2 was repeated except that 3-hydroxyphenylacetonitrile was used in place of 4-ethoxycarbonyl-4-(3-hydroxyphenyl)tetrahydropyran. There was thus obtained the required starting material in 63% yield, m.p. 145-146°C.
IR Spectrum 2240 cm⁻¹.

The 3-hydroxyphenylacetonitrile used above was obtained as follows:-
A mixture of m-cresol (10.8 g), tert-butyldimethylsilyl chloride (15 g), imidazole (6.8 g) and DMF (100 ml) was stirred at ambient temperature for 5 hours. The mixture was partitioned between diethyl ether and water. The organic phase was washed with 2N aqueous sodium hydroxide solution and brine, dried (MgSO₄) and evaporated. There was thus obtained 3-(tert-butyldimethylsilyloxy)toluene (18.2 g, 82%) as a liquid.

A mixture of the product so obtained (17.2 g), N-bromosuccinimide (16.54 g), 2,2'-azobisisobutyronitrile (1.7 g) and carbon tetrachloride (170 ml) was heated to reflux and irradiated with the light from a 250 Watt lamp for 30 minutes. The mixture was cooled to ambient temperature and filtered. The filtrate was evaporated to give 3-(tert-butyldimethylsilyloxy)benzyl bromide as a liquid which was used directly. Thus the liquid was dissolved in methylene chloride (150 ml) and the solution was cooled to 0°C. Tetrabutylammonium cyanide (24.6 g) was added and the mixture was stirred at 0°C for 1.5 hours and at ambient temperature for 2.5 hours. The mixture was evaporated and the residue was purified by column chromatography using increasingly polar mixtures of methylene chloride and petroleum ether (b.p. 40-60°C) as eluent. There was thus obtained 3-(tert-butyldimethylsilyloxy)phenylacetonitrile (8 g, 42%) as an oil.

Tetrabutylammonium fluoride (1.6M in THF, 15 ml) was added to a solution of the acetonitrile so obtained (4 g) in THF (15 ml) and the mixture was stirred at ambient temperature for 75 minutes. The mixture was evaporated and the residue was partitioned between ethyl acetate and water. The organic phase was washed with water and brine, dried (MgSO₄) and evaporated. There was thus obtained 3-hydroxyphenylacetonitrile (1.85 g, 86%), IR Spectrum 2280 cm⁻¹.

### EXAMPLE 5

Using the procedure described in the third paragraph of the portion of Example 2 which is concerned with the preparation of 4-ethoxycarbonyl-4-(3-hydroxyphenyl)tetrahydropyran, 3-(7-fluoronaphth-2-ylmethoxy)phenylacetonitrile was reacted with bis-2-chloroethyl ether to give 4-cyano-4-[3-(7-fluoronaphth-2-ylmethoxy)phenyl]tetrahydropyran in 39% yield, m.p. 106-107°C.
IR Spectrum 2215 cm⁻¹.

The 3-(7-fluoronaphth-2-ylmethoxy)phenylacetonitrile used as a starting material was obtained as follows:-
Using the procedure described in Example 2, 2-bromomethyl-7-fluoronaphthalene was reacted with 3-hydroxyphenylacetonitrile to give the required starting material in 70% yield, m.p. 86-87°C,
IR Spectrum 2240 cm⁻¹.

### EXAMPLE 6

The procedure described in Example 2 was repeated except that 4-hydroxymethyl-4-(3-hydroxyphenyl)tetrahydropyran was used in place of 4-ethoxycarbonyl-4-(3-hydroxyphenyl)tetrahydropyran. There was thus obtained 4-hydroxymethyl-4-[3-(1-methyl-2-oxo-1,2-dihydroquinolin-6-ylmethoxy)phenyl]tetrahydropyran in 71% yield, m.p. 61°C.

The 4-hydroxymethyl-4-(3-hydroxyphenyl)tetrahydropyran used as a starting material was obtained as follows:-
A solution of ethyl 3-benzyloxyphenylacetate (0.45 g) in diethyl ether (2 ml) was added to a stirred suspension of lithium aluminium hydride (0.17 g) in diethyl ether (15 ml) and the mixture was stirred at ambient temperature for 10 minutes. Water (10 ml) was cautiously added dropwise to destroy the excess of reducing agent. The mixture was extracted with diethyl ether. The organic phase was dried (MgSO₄) and evaporated. There was thus obtained 4-(3-benzyloxyphenyl)-4-hydroxymethyltetrahydropyran (0.375 g) as an oil.

A mixture of the product so obtained, 10% palladium-on-charcoal catalyst (0.04 g) and ethanol (5 ml) was stirred under two atmospheres of hydrogen for 8 hours. The mixture was filtered and the filtrate was evaporated. The residue was purified by column chromatography using increasingly polar mixtures of methylene chloride and acetone as eluent. There was thus obtained the required starting material (0.212 g, 80%), m.p. 115°C.

### EXAMPLE 7

Sodium hydride (60% w/w dispersion in mineral oil, 0.017 g) was added to a mixture of 4-hydroxymethyl-4-[3-(1-methyl-2-oxo-1,2-dihydroquinolin-6-ylmethoxy)phenyl]tetrahydropyran (0.1 g), 15-crown-5 (2 drops) and DMF (1.5 ml) and the mixture was stirred at ambient temperature for 10 minutes. Methyl iodide (0.041 ml) was added and the mixture was stirred at ambient temperature for 12 hours. The mixture was partitioned between ethyl acetate and water. The organic phase was washed with brine, dried (MgSO₄) and evaporated. The residue was purified by column chromatography using increasingly polar mixtures of methylene chloride and acetone as eluent. There was thus obtained 4-methoxymethyl-4-[3-(1-methyl-2-oxo-1,2-dihydroquinolin-6-ylmethoxy)phenyl]tetrahydropyran (0.072 g, 70%), m.p. 139-140°C.

### EXAMPLE 8

Using a similar procedure to that described in Example 1, 4-mercapto-4-[3-(naphth-2-ylmethoxy)phenyl]tetrahydropyran was reacted with isopropyl iodide to give 4-isopropylthio-4-[3-(naphth-2-ylmethoxy)phenyl]tetrahydropyran in 67% yield, as an oil.
NMR Spectrum (CDCl₃, δ values) 0.9(d, 6H), 2.0-2.4(m, 5H), 3.6-4.0(m, 4H), 5.23(s, 2H), 6.85(m, 1H), 7.1(m, 2H), 7.3(m, 1H), 7.5(m, 3H), 7.9(m, 4H).

### EXAMPLE 9

Using a similar procedure to that described in Example 1, 4-mercapto-4-[3-(naphth-2-ylmethoxy)phenyl]tetrahydropyran was reacted with 2,2,2-trifluoroethyl iodide to give 4-[3-(naphth-2-ylmethoxy)phenyl]-4-(2,2,2-trifluoroethylthio)tetrahydropyran in 61% yield, m.p. 76-78°C.

### EXAMPLE 10

3-Chloroperoxybenzoic acid (60% purity, 0.24 g) was added portionwise to a stirred solution of 4-methylthio-4-[3-(naphth-2-ylmethoxy)phenyl]tetrahydropyran (0.276 g) in chloroform (5 ml) and the mixture was stirred at ambient temperature for 30 minutes. Calcium hydroxide (0.293 g) was added and the mixture was stirred at ambient temperature for 30 minutes. The mixture was filtered and the filtrate was evaporated. The residue was purified by column chromatography using a 39:1 v/v mixture of dichloromethane and methanol as eluent. There was thus obtained 4-methylsulphinyl-4-[3-(naphth-2-ylmethoxy)phenyl]tetrahydropyran (0.133 g, 50%), as an oil.
NMR Spectrum (CDCl₃, δ values) 1.84(s, 3H), 2.05-2.5(m, 4H), 3.4-4.1(m, 4H), 5.24(s, 2H), 7.0-7.9(m, 11H).

### EXAMPLE 11

A mixture of 6-bromomethyl-1-methyl-1,2-dihydroquinolin-2-one (0.36 g), 4-(5-fluoro-3-hydroxyphenyl)-4-methylthiotetrahydropyran (0.363 g), potassium carbonate (0.55 g) and acetone (20 ml) was stirred and heated to reflux for 90 minutes. The mixture was concentrated by evaporation of most of the solvent. The residue was partitioned between ethyl acetate and water. The organic phase was washed with water, dried (MgSO₄) and evaporated. The residue was purified by column chromatography using a 1:1 v/v mixture of hexane and ethyl acetate as eluent. There was thus obtained 4-[5-fluoro-3-(1-methyl-2-oxo-1,2-dihydroquinolin-6-ylmethoxy)phenyl]-4- methylthiotetrahydropyran (0.37 g, 60%), m.p. 118-120°C.

The 4-(5-fluoro-3-hydroxyphenyl)-4-methylthiotetrahydropyran, used as a starting material, was obtained as follows:
Sodium hydride (50% w/w dispersion in mineral oil, 12.4 g) was added portionwise to a mixture of benzyl alcohol (26.7 ml) and DMA (500 ml) and the mixture was stirred at ambient temperature for 1 hour. 1-Bromo-3,5-difluorobenzene (50 g) was added carefully to control the vigour of the ensuing exothermic reaction. The mixture was stirred at ambient temperature for 2 hours and the solvent was evaporated. The residue was partitioned between methylene chloride and water and the organic phase was washed with water (4 x 50 ml), dried (MgSO₄) and evaporated. The residue was purified by distillation to give 3-benzyloxy-1-bromo-5-flurobenzene (41.8 g, 57%), as a colourless liquid (b.p. 124-130°C at 0.3 mm Hg).

A solution of a portion (9.75 g) of this product in THF (150 ml) was cooled to -75°C and n-butyl-lithium (1.6 M in hexane, 22 ml) was added dropwise. The mixture was stirred at -75°C for 1 hour and a solution of tetrahydropyran-4-one (3.47 g) in THF (10 ml) was added dropwise. The mixture was stirred at -75°C for 1 hour and then allowed to warm to 0°C. A saturated aqueous ammonium chloride solution (50 ml) was added and the organic phase was separated, dried (MgSO₄) and evaporated. The residue was purified by column chromatography using a 1:1 v/v mixture of toluene and ethyl acetate as eluent. There was thus obtained 4-(3-benzyloxy-5-fluorophenyl)-4-hydroxytetrahydropyran (7.4 g, 71%) as an oil.

Trifluoromethanesulphonic acid (0.2 ml) was added dropwise to a solution of 4-(3-benzyloxy-5-fluorophenyl)-4-hydroxytetrahydropyran (1.2 g) in methanethiol (10 ml) and the mixture was stirred at ambient temperature for 10 minutes. Boron trifluoride etherate (5.6 ml) was added and the mixture was stirred at ambient temperature for 1 hour. The mixture was partitioned between water and diethyl ether. The organic phase was washed with water and with a saturated aqueous sodium bicarbonate solution, dried (MgSO₄) and evaporated. The residue was purified by column chromatography using a 3:1 v/v mixture of hexane and ethyl acetate as eluent. There was thus obtained the required starting material (0.83 g, 86%), m.p. 120-123°C.

### EXAMPLE 12

Using a similar procedure to that described in Example 11, 6-bromomethyl-1-methyl-1,2-dihydroquinolin-2-one was reacted with 4-ethylthio-4-(5-fluoro-3-hydroxyphenyl)tetrahydropyran to give 4-ethylthio-4-[5-fluoro-3-(1-methyl-2-oxo-1,2-dihydroquinolin-6-ylmethoxy)phenyl]tetrahydropyran in 40% yield, m.p. 73-75°C.

The 4-ethylthio-4-(5-fluoro-3-hydroxyphenyl)tetrahydropyran, used as a starting material, was obtained by repetition of the procedure described in the last paragraph of the portion of Example 11 which is concerned with the preparation of starting materials except that ethanethiol was used in place of methanethiol. There was thus obtained the required starting material in 90% yield, m.p. 68-72°C.

### EXAMPLE 13

Using a similar procedure to that described in Example 11, 6-bromomethyl-1-methyl-1,2-dihydroquinolin-2-one was reacted with 4-tert-butylthio-4-(5-fluoro-3-hydroxyphenyl)tetrahydropyran to give 4-tert-butylthio-4-[5-fluoro-3-(1-methyl-2-oxo-1,2-dihydroquinolin-6-ylmethoxy)phenyl]tetrahydropyran in 30% yield, m.p. 54-56°C.

The 4-tert-butylthio-4-(5-fluoro-3-hydroxyphenyl)tetrahydropyran, used as a starting material, was obtained by repetition of the procedure described in the last paragraph of the portion of Example 11 which is concerned with the preparation of starting materials except that tert-butanethiol was used in place of methanethiol. There was thus obtained the required starting material in 13% yield, as an oil.
NMR Spectrum (CDCl₃, δ values) 1.05(s, 9H), 2.2(t, 4H), 3.6-4.1(m, 4H), 6.5(m, 1H), 6.9(m, 2H).

### EXAMPLE 14

A mixture of 6-bromomethyl-1-methyl-1,2-dihydroquinolin-2-one (0.34 g), 4-(1-hydroxyethyl)-4-(3-hydroxyphenyl)tetrahydropyran (0.3 g), potassium carbonate (0.205 g) and DMF (3 ml) was stirred at ambient temperature for 16 hours. The mixture was partitioned between ethyl acetate and water. The organic phase was washed with water and with brine, dried (MgSO₄) and evaporated. The residue was purified by column chromatography using ethyl acetate as eluent. There was thus obtained an oil which crystallised upon trituration in a mixture of pentane and diethyl ether. There was thus obtained 4-(1-hydroxyethyl)-4-[3-(1-methyl-2-oxo-1,2-dihydroquinolin-6-ylmethoxy)phenyl]tetrahydropyran (0.366 g), 69%), m.p. 59-60°C.

The 4-(1-hydroxyethyl)-4-(3-hydroxyphenyl)tetrahydropyran, used as a starting material, was obtained as follows:-
A solution of oxalyl chloride (1.463 ml) in methylene chloride (5 ml) was added dropwise to a stirred mixture of DMSO (2.38 ml) and methylene chloride (5 ml) which had been cooled to -70°C. The mixture was stirred at -65°C for 15 minutes. A solution of 4-(3-benzyloxyphenyl)-4-hydroxymethyltetrahydropyran (5 g) in methylene chloride (5 ml) was added dropwise to the mixture which was maintained at a temperature of -60°C. The mixture was stirred at -60°C for 1 hour. Triethylamine (11.7 ml) was added and the mixture was stirred at -60°C for 1.5 hours. The mixture was allowed to warm to ambient temperature and stirred for a further 1.5 hours. The mixture was partitioned between methylene chloride and water. The organic phase was washed with water and with brine, dried (MgSO₄) and evaporated. The residue was purified by column chromatography using methylene chloride as eluent. There was thus obtained 4-(3-benzyloxyphenyl)-4-formyltetrahydropyran (3.57 g, 72%) as a solid.

Methylmagnesium bromide (3M in diethyl ether, 2 ml) was added dropwise to a solution of a portion (1.184 g) of the 4-formyltetrahydropyran so obtained in THF (20 ml) and the mixture was stirred at ambient temperature for 16 hours. The mixture was evaporated and the residue was partitioned between methylene chloride and a saturated aqueous ammonium chloride solution. The organic phase was washed with water and with brine, dried (MgSO₄) and evaporated. The residue was purified by column chromatography using initially methylene chloride and then a 5:1 v/v mixture of methylene chloride and diethyl ether as eluent. There was thus obtained 4-(3-benzyloxyphenyl)-4-(1-hydroxyethyl)tetrahydropyran (0.96 g, 77%), m.p. 119°C.

A mixture of a portion (0.8 g) of the product so obtained, 10% palladium-on-charcoal catalyst (0.25 g), ethyl acetate (5 ml) and ethanol (10 ml) was stirred under four atmospheres of pressure of hydrogen for 16 hours. The mixture of filtered and the filtrate was evaporated. The residue was purified by column chromatography using initially methylene chloride and then a 4:1 v/v mixture of methylene chloride and acetone as eluent. There was thus obtained 4-(1-hydroxyethyl)-4-(3-hydroxyphenyl)tetrahydropyran (0.472 g, 78%), m.p. 113°C.

### EXAMPLE 15

Using a similar procedure to that described in Example 14, 6-bromomethyl-1-methyl-1,2-dihydroquinolin-2-one was reacted with 4-formyl-4-(3-hydroxyphenyl)tetrahydropyran to give 4-formyl-4-[3-(1-methyl-2-oxo-1,2-dihydroquinolin-6-ylmethoxy)phenyl]tetrahydropyran in 45% yield as a glassy solid.
NMR Spectrum (CDCl₃, δ values) 1.9-2.85(m, 4H), 3.98(s, 3H), 3.4-4.1(m, 4H), 5.11(s, 2H), 6.65-7.10(m, 4H), 7.25-7.8(m, 5H), 9.4(s, 1H).

The 4-formyl-4-(3-hydroxyphenyl)tetrahydropyran, used as a starting material, was obtained as follows:-
A mixture of 4-(3-benzyloxyphenyl)-4-formyltetrahydropyran (1 g), 10% palladium-on-charcoal catalyst (0.15 g), ethyl acetate (10 ml) and ethanol (20 ml) was stirred under 2.7 atmospheres pressure of hydrogen for 16 hours. The mixture was filtered and the filtrate was evaporated. The residue was purified by column chromatography using initially methylene chloride and then a 20:3 v/v mixture of methylene chloride and diethyl ether as eluent. There was thus obtained the required starting material (0.162 g, 23%), m.p. 123-124°C.

### EXAMPLE 16

Using a similar procedure to that described in Example 14, 6-bromomethyl-1-methyl-1,2-dihydroquinolin-2-one was reacted with 4-acetyl-4-(3-hydroxyphenyl)tetrahydropyran to give 4-acetyl-4-[3-(1-methyl-2-oxo-1,2-dihydroquinolin-6-ylmethoxy)phenyl]tetrahydropyran in 52% yield, m.p. 125-126°C.

The 4-acetyl-4-(3-hydroxyphenyl)tetrahydropyran, used as a starting material, was obtained as follows:-
The procedure described in the first paragraph of the portion of Example 14 which is concerned with the preparation of starting materials was repeated except that 4-(1-hydroxyethyl)-4-(3-hydroxyphenyl)tetrahydropyran was used in place of 4-(3-benzyloxyphenyl)-4-hydroxymethyltetrahydropyran. There was thus obtained the required starting material in 77% yield, m.p. 120-121°C.

### EXAMPLE 17

Using a similar procedure to that described in Example 14, 6-bromomethyl-1-methyl-1,2-dihydroquinolin-2-one was reacted with 4-(5-fluoro-3-hydroxyphenyl)-3,4,5,6-tetrahydro-2H-pyran to give 4-[5-fluoro-3-(1-methyl-2-oxo-1,2-dihydroquinolin-6-ylmethoxy)phenyl]-3,4,5,6-tetrahydro-2H-pyran in 46% yield, m.p. 126-127°C.

The 4-(5-fluoro-3-hydroxyphenyl)-3,4,5,6-tetrahydro-2H-pyran, used as a starting material, was obtained as follows:-
Sodium hydride (50% w/w dispersion in mineral oil, 2.11 g) was added portionwise to a stirred solution of 4-(3-benzyloxy-5-fluorophenyl)-4-hydroxytetrahydropyran (12.1 g) in THF (150 ml). The mixture was stirred at ambient temperature for 1 hour, cooled in an ice-bath and methyl iodide (3.75 ml) was added dropwise. The mixture was stirred at ambient temperature for 18 hours, 2N aqueous hydrochloric acid (3 drops) were added and the organic solvent was evaporated. The residue was partitioned between ethyl acetate and water. The organic phase was separated, washed with water and with brine, dried (MgSO₄) and evaporated. There was thus obtained 4-(3-benzyloxy-5-fluorophenyl)-4-methoxytetrahydropyran (12.5 g, 99%), as a pale yellow oil which was used without further purification.

A solution of the product so obtained in ethanol (100 ml) was hydrogenated in the presence of 10% palladium-on-charcoal catalyst for 3 hours. The mixture was filtered and the filtrate was evaporated. There was thus obtained 4-(5-fluoro-3-hydroxyphenyl)-4-methoxytetrahydropyran (7.7 g, 86%), m.p. 123-124°C.

A saturated solution of hydrogen chloride in diethyl ether (1.5 ml) was added to a solution of a portion (0.678 g) of the product so obtained in methanol (15 ml). The mixture was heated to reflux for 20 hours. The mixture was evaporated and the residue was purified by column chromatography using a 2:1 v/v mixture of hexane and ethyl acetate as eluent. There was thus obtained 4-(5-fluoro-3-hydroxyphenyl)-3,6-dihydro-2H-pyran (0.417 g, 72%), m.p. 137-138°C.

A mixture of a portion (0.334 g) of the product so obtained, 10% palladium-on-charcoal catalyst (0.065 g) and ethanol (15 ml) was stirred under an atmosphere of hydrogen for 7 hours. The mixture was filtered and the filtrate was evaporated. The residue was purified by column chromatography using diethyl ether as eluent. There was thus obtained the required starting material (0.311 g, 92%), m.p. 128-130°C.

### EXAMPLE 18

Using a similar procedure to that described in Example 14, 6-bromomethyl-1-methyl-1,2-dihydroquinolin-2-one was reacted with 4-(3-hydroxyphenyl)-4-methyltetrahydropyran to give 4-methyl-4-[3-(1-methyl-2-oxo-1,2-dihydroquinolin-6-ylmethoxy)phenyl]tetrahydropyran in 40% yield, m.p. 107-108°C.

The 4-(3-hydroxyphenyl)-4-methyltetrahydropyran, used as a starting material, was obtained as follows:-
A mixture of iodine (1.91 g), imidazole (0.68 g), triphenylphosphine (1.97 g), acetonitrile (5 ml) and bis(2-methoxyethyl ether (15 ml) was stirred at ambient temperature for 1 hour. A solution of 4-(3-benzyloxyphenyl)-4-hydroxymethyltetrahydropyran (1.5 g) in acetonitrile (2 ml) was added and the mixture was heated to 110°C for 6 hours. The mixture was evaporated and the residue was partitioned between ethyl acetate and an aqueous sodium dithionite solution (10% w/v). The organic phase was washed with water and with brine, dried (MgSO₄) and evaporated. The residue was purified by column chromatography using a 2:1 v/v mixture of petroleum ether (b.p. 40-60°C) and methylene chloride as eluent. There was thus obtained an oil which crystallised on trituration under pentane. There was thus obtained 4-(3-benzyloxyphenyl)-4-iodomethyltetrahydropyran (1.43 g, 70%), m.p. 77°C.

A mixture of a portion (0.58 g) of the product so obtained, triethylamine (0.395 ml), 10% palladium-on-charcoal catalyst (0.2 g) and ethyl acetate (6 ml) was stirred under 4.7 atmospheres pressure of hydrogen for 16 hours. The mixture was filtered and the filtrate was evaporated. The residue was purified by column chromatography using a 6:1 v/v mixture of petroleum ether (b.p. 40-60°C) and ethyl acetate as eluent. There was thus obtained 4-(3-benzyloxyphenyl)-4-methyltetrahydropyran (0.22 g, 55%), as an oil.

A mixture of the product so obtained, 10% palladium-on-charcoal catalyst (0.4 g) and ethanol (5 ml) was stirred under 4 atmospheres pressure of hydrogen for 5 hours. The mixture was filtered and the filtrate was evaporated. The residue was purified by column chromatography using a 10:1 v/v mixture of methylene chloride and diethyl ether as eluent. There was thus obtained the required starting material (0.14 g, 92%), m.p. 94-95°C.

### EXAMPLE 19

Using a similar procedure to that described in Example 14, 6-bromomethyl-1-methyl-1,2-dihydroquinolin-2-one was reacted with 4-ethyl-4-(3-hydroxyphenyl)tetrahydropyran to give 4-ethyl-4-[3-(1-methyl-2-oxo-1,2-dihydroquinolin-6-ylmethoxy)phenyl]tetrahydropyran in 63% yield, m.p. 139°C.

The 4-ethyl-4-(3-hydroxyphenyl)tetrahydropyran, used a starting material, was obtained as follows:-
n-Butyl-lithium (1.6M in hexane, 1.375 ml) was added dropwise to a solution of methyltriphenylphosphonium bromide (0.786 g) in a mixture of THF (15 ml) and diethyl ether (2 ml). The mixture was stirred at ambient temperature for 2 hours. The solution so obtained was added dropwise to a solution of 4-(3-benzyloxyphenyl)-4-formyltetrahydropyran (0.592 g) in diethyl ether (10 ml) and the mixture was stirred at ambient temperature for 4 hours. The mixture was filtered and the filtrate was evaporated. The residue was purified by column chromatography using increasingly polar mixtures of petroleum ether (b.p 40-60°C) and ethyl acetate as eluent. There was thus obtained 4-(3-benzyloxyphenyl)-4-vinyltetrahydropyran (0.407 g, 70%), as an oil.

A mixture of the product so obtained, 10% palladium-on-charcoal catalyst (0.05 g) and ethanol (5 ml) was stirred under 3 atmospheres pressure of hydrogen for 16 hours. The mixture was filtered and the filtrate was evaporated. The residue was purified by column chromatography using initially methylene chloride and then a 10:1 v/v mixture of methylene chloride and diethyl ether as eluent. There was thus obtained the required starting material (0.27 g, 96%), m.p. 64-65°C.

### EXAMPLE 20

Using a similar procedure to that described in Example 14, 6-bromomethyl-1-methyl-1,2-dihydroquinolin-2-one was reacted with (2RS,4RS)-4-ethyl-4-(5-fluoro-3-hydroxyphenyl)-2-methyltetrahydropyran to give (2RS,4RS)-4-ethyl-4-[5-fluoro-3-(1-methyl-2-oxo-1,2-dihydroquinolin-6-ylmethoxy)phenyl]-2-methyltetrahydropyran in 52% yield, m.p. 99°C.

The (2RS,4RS)-4-ethyl-4-(5-fluoro-3-hydroxyphenyl)-2-methyltetrahydropyran, used as a starting material, was obtained as follows:-
A mixture of iodine (115.5 g), imidazole (41.2 g), triphenylphosphine (159 g), acetonitrile (200 ml) and diethyl ether (500 ml) was stirred at ambient temperature for 30 minutes. A solution of 2-methyl-3-oxapentane-1,5-diol (J.C.S. Perkin I, 1979, 1029; 18.2 g) in diethyl ether (20 ml) was added dropwise and the mixture was stirred at ambient temperature for 3 hours. The mixture was poured into petroleum ether (b.p. 40-60°C, 3 litres). The organic solution was decanted from the residue which had been deposited. The organic solution was evaporated and the material so obtained was purified by column chromatography using initially petroleum ether (b.p. 40-60°C) and then a 10:1 v/v mixture of petroleum ether (b.p. 40-60°C) and methylene chloride as eluent. There was thus obtained 1,5-diiodo-2-methyl-3-oxapentane (30.3 g, 61%) as a liquid.

Sodium hydride (60% w/w dispersion in mineral oil, 9.17 g) was added portionwise to a solution of ethyl 3,5-difluorophenylacetate (17 g) in THF (400 ml) and the mixture was stirred at ambient temperature for 1 hour. A solution of 1,5-diiodo-2-methyl-3-oxapentane (30.3 g) in THF (20 ml) was added dropwise and the mixture was stirred at ambient temperature for 2 hours. The mixture was filtered and the filtrate was evaporated. The residue was purified by column chromatography using a 19:1 v/v mixture of petroleum ether (b.p. 40-60°C) and ethyl acetate as eluent. There was thus obtained, as a mixture of diastereoisomers, 4-ethoxycarbonyl-4-(3,5-difluorophenyl)-2-methyltetrahydropyran (16.5 g). The mixture of diastereoisomers was separated by further chromatography using a 10:1 v/v mixture of petroleum ether (b.p 40-60°C) and ethyl acetate as eluent. There was thus obtained:-
a less polar isomer (7.75 g), the (2RS,4SR)-isomer, having the 2-methyl and 4-ethoxycarbonyl groups in a trans-relationship; and a more polar isomer (5.9 g), the (2RS,4RS)-isomer, having the 2-methyl an 4-ethoxycarbonyl groups in a cis-relationship.

Sodium hydride (60% w/w dispersion in mineral oil, 0.258 g) was added portionwise to a stirred solution of benzyl alcohol (0.582 g) in N-methylpyrrolidin-2-one (10 ml) and the mixture was stirred at ambient temperature for 45 minutes. A solution of a portion (1.53 g) of the less polar isomer obtained above in N-methylpyrrolidin-2-one (5 ml) was added and the mixture was stirred at ambient temperature for 1.5 hours and then heated to 50°C for 1.5 hours. The mixture was partitioned between diethyl ether and water. The organic phase was washed with water and with brine, dried (MgSO₄) and evaporated. The residue was purified by column chromatography using a 10:1 v/v mixture of petroleum ether (b.p 40-60°C) and ethyl acetate as eluent. There was thus obtained (2RS,4SR)-4-(3-benzyloxy-5-fluorophenyl)-4-ethoxycarbonyl-2-methyltetrahydropyran (0.756 g, 45%), as an oil.

A solution of the material so obtained in diethyl ether (4 ml) was added to a stirred suspension of lithium aluminium hydride (0.07 g) in diethyl ether (10 ml) and the mixture was stirred at ambient temperature for 30 minutes. Water (5 ml) was added cautiously to destroy the excess of reducing agent. The mixture was extracted with diethyl ether. The organic phase was washed with water, dried (MgSO₄) and evaporated. The residue was purified by column chromatography using a 10:3 v/v mixture of petroleum ether (b.p. 40-60°C) and ethyl acetate as eluent. There was thus obtained (2RS,4SR)-4-(3-benzyloxy-5-fluorophenyl)-4-hydroxymethyl-2-methyltetrahydropyran (0.589 g, 89%), as an oil.

Using a similar procedure to that described in the first paragraph of the portion of Example 14 which is concerned with the preparation of starting materials, the 4-hydroxymethyltetrahydropyran so obtained was oxidised to give (2RS,4SR)-4-(3-benzyloxy-5-fluorophenyl)-4-formyl-2-methyltetrahydropyran (0.524 g, 91%), as an oil.

n-Butyl-lithium (1.6 M in hexane, 1.16 ml) was added dropwise to a solution of methyltriphenylphosphonium bromide (0.664 g) in THF (10 ml) which had been cooled to -70°C. The mixture was stirred at -70°C for 1 hours. A solution of the 4-formyltetrahydropyran so obtained in THF (2 ml) was added dropwise and the mixture was stirred at -70°C for 30 minutes. The mixture was allowed to warm to ambient temperature. The mixture was filtered and the filtrate was evaporated. The residue was purified by column chromatography using methylene chloride as eluent. There was thus obtained (2RS,4RS)-4-(3-benzyloxy-5-fluorophenyl)-2-methyl-4-vinyltetrahydropyran (0.4 g, 79%), as an oil.

Using a similar procedure to that described in the last paragraph of the portion of Example 19 which is concerned with the preparation of starting materials, the 4-vinyltetrahydropyran so obtained was hydrogenated to give (2RS,4RS)-4-ethyl-4-(5-fluoro-3-hydroxyphenyl)-2-methyltetrahydropyran (0.26 g, 92%), as an oil.
NMR Spectrum (CDCl₃, δ values) 0.57(t, 3H), 1.20(d, 3H), 1.15-2.10(m, 6H), 3.5-4.1(m, 3H), 5.10(s, 1H), 6.3-6.7(m, 3H).

### EXAMPLE 21

Using a similar procedure to that described in Example 14, 6-bromomethyl-1-methyl-1,2-dihydroquinolin-2-one was reacted with (2RS,4SR)-4-ethyl-4-(5-fluoro-3-hydroxyphenyl)-2-methyltetrahydropyran to give (2RS,4SR)-4-ethyl-4-[5-fluoro-3-(1-methyl-2-oxo-1,2-dihydroquinolin-6-ylmethoxy)phenyl]-2-methyltetrahydropyran in 68% yield, as a foam.
NMR Spectrum (CDCl₃, δ values) 0.5(t, 3H), 1.1(d, 3H), 1.1-2.2(m, 6H), 3.1-4.0(m, 6H), 5.05(s, 2H), 6.4-6.7(m, 4H), 7.2-7.7(m, 4H).

The (2RS,4SR)-4-ethyl-4-(5-fluoro-3-hydroxyphenyl)-2-methyltetrahydropyran, used as a starting material, was obtained as follows:-
The procedures described in the last five paragraphs of the portion of Example 20 which is concerned with the preparation of starting materials were repeated except that the more polar diasteroisomer, i.e. (2RS,4SR)-4-ethoxycarbonyl-4-(3,5-difluorophenyl)-2-methyltetrahydropyran, was used in place of the less polar diastereoisomer. There was thus obtained the required starting material in 14% yield, as an oil.
NMR Spectrum (CDCl₃, δ values) 0.6(t, 3H), 1.15(d, 3H), 1.2-2.4(m, 6H), 3.25-4.0(m, 3H), 5.4(broad s, 1H), 6.3-6.6(m, 3H).

### EXAMPLE 22

The following illustrate representative pharmaceutical dosage forms containing the compound of formula I, or a pharmaceutically-acceptable salt thereof (hereafter compound X), for therapeutic or prophylactic use in humans:
(a)

| Tablet I | mg/tablet |
|---|---|
| Compound X | 100 |
| Lactose Ph.Eur | 182.75 |
| Croscarmellose sodium | 12.0 |
| Maize starch paste (5% w/v paste) | 2.25 |
| Magnesium stearate | 3.0 |

(b)

| Tablet II | mg/tablet |
|---|---|
| Compound X | 50 |
| Lactose Ph.Eur | 223.75 |
| Croscarmellose sodium | 6.0 |
| Maize starch | 15.0 |
| Polyvinylpyrrolidone (5% w/v paste) | 2.25 |
| Magnesium stearate | 3.0 |

(c)

| Tablet III | mg/tablet |
|---|---|
| Compound X | 1.0 |
| Lactose Ph.Eur | 93.25 |
| Croscarmellose sodium | 4.0 |
| Maize starch paste (5% w/v paste) | 0.75 |
| Magnesium stearate | 1.0 |

(d)

| Capsule | mg/capsule |
|---|---|
| Compound X | 10 |
| Lactose Ph.Eur | 488.5 |
| Magnesium stearate | 1.5 |

(e)

| Injection I | (50 mg/ml) |
|---|---|
| Compound X | 5.0% w/v |
| 1M Sodium hydroxide solution | 15.0% v/v |
| 0.1M Hydrochloric acid (to adjust pH to 7.6) | |
| Polyethylene glycol 400 | 4.5% w/v |
| Water for injection to 100% | |

(f)

| Injection II | (10 mg/ml) |
|---|---|
| Compound X | 1.0% w/v |
| Sodium phosphate BP | 3.6% w/v |
| 0.1M Sodium hydroxide solution | 15.0% v/v |
| Water for injection to 100% | |

(g)

| Injection III | (1mg/ml,buffered to pH6) |
|---|---|
| Compound X | 0.1% w/v |
| Sodium phosphate BP | 2.26% w/v |
| Citric acid | 0.38% w/v |
| Polyethylene glycol 400 | 3.5% w/v |
| Water for injection to 100% | |

(h)

| Aerosol I | mg/ml |
|---|---|
| Compound X | 10.0 |
| Sorbitan trioleate | 13.5 |
| Trichlorofluoromethane | 910.0 |
| Dichlorodifluoromethane | 490.0 |

(i)

| Aerosol II | mg/ml |
|---|---|
| Compound X | 0.2 |
| Sorbitan trioleate | 0.27 |
| Trichlorofluoromethane | 70.0 |
| Dichlorodifluoromethane | 280.0 |
| Dichlorotetrafluoroethane | 1094.0 |

(j)

| Aerosol III | mg/ml |
|---|---|
| Compound X | 2.5 |
| Sorbitan trioleate | 3.38 |
| Trichlorofluoromethane | 67.5 |
| Dichlorodifluoromethane | 1086.0 |
| Dichlorotetrafluoroethane | 191.6 |

(k)

| Aerosol IV | mg/ml |
|---|---|
| Compound X | 2.5 |
| Soya lecithin | 2.7 |
| Trichlorofluoromethane | 67.5 |
| Dichlorodifluoromethane | 1086.0 |
| Dichlorotetrafluoroethane | 191.6 |

### Note

The above formulations may be obtained by conventional procedures well known in the pharmaceutical art. The tablets (a)-(c) may be enteric coated by conventional means, for example to provide a coating of cellulose acetate phthalate. The aerosol formulations (h)-(k) may be used in conjunction with standard, metered dose aerosol dispensers, and the suspending agents sorbitan trioleate and soya lecithin may be replaced by an alternative suspending agent such as sorbitan monooleate, sorbitan sesquioleate, polysorbate 80, polyglycerol oleate or oleic acid.

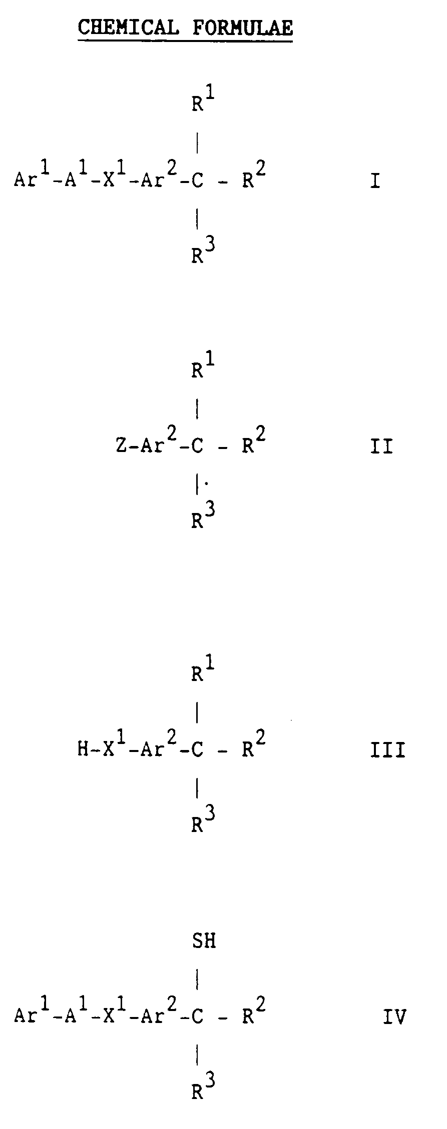

## Claims

1. A cyclic ether derivative of the formula I 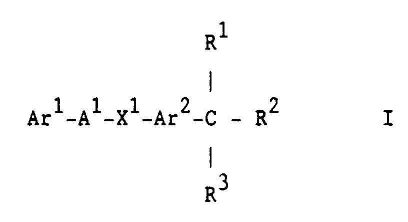 wherein Ar¹ is phenyl or naphthyl, or a 9- or 10-membered bicyclic heterocyclic moiety containing one or two nitrogen heteroatoms and optionally containing a further heteroatom selected from nitrogen, oxygen and sulphur, and Ar¹ may optionally bear up to five substituents selected from amino, halogeno, hydroxy, cyano, oxo, thioxo, (1-4C)alkyl, (1-4C)alkoxy, (1-4C)alkylthio, (1-4C)alkylsulphinyl, (1-4C)alkylsulphonyl, (1-4C)alkylamino, di-[(1-4C)alkyl]amino, (2-4C)alkanoyl, fluoro-(1-4C)alkyl, cyano-(1-4C)alkyl, phenyl, benzoyl and phenyl-(1-4C)alkyl, and wherein said phenyl, benzoyl or phenyl-(1-4C)alkyl substituents may optionally bear a substituent selected from halogeno, (1-4C)alkyl and (1-4C)alkoxy;
wherein A¹ is a direct link to X¹ or is (1-3C)alkylene;
wherein X¹ is oxy, thio, sulphinyl, sulphonyl or imino;
wherein Ar² is phenylene which may optionally bear one or two substituents selected from halogeno, hydroxy, amino, nitro, cyano carbamoyl, ureido, (1-4C)alkyl, (1-4C)alkoxy, (1-4C)alkylamino, di-[(1-4C)alkyl]amino, fluoro-(1-4C)alkyl and (2-4C)alkanoylamino; or Ar² is pyridylene;
wherein R¹ is (1-4C)alkylthio, (1-4C)alkylsulphinyl, (1-4C)alkylsulphonyl, (1-4C)alkylamino or di-[(1-4C)alkyl]amino, or R¹ is hydrogen, formyl, cyano, carbamoyl, (1-4C)alkyl, (1-4C)alkoxycarbonyl, N-(1-4C)alkylcarbamoyl, N,N-di-[(1-4C)alkyl]carbamoyl, hydroxy-(1-4C)alkyl, fluoro-(1-4C)alkylthio, (2-4C)alkanoyl or (1-4C)alkoxy-(1-4C)alkyl; and
wherein R² and R³ together form a group of the formula -A²-X²-A³- which, together with the carbon atom to which A² and A³ are attached, defines a ring having 5 to 7 ring atoms, wherein A² and A³, which may be the same or different, each is (1-3C)alkylene and X² is oxy, thio, sulphinyl or sulphonyl, and which ring may bear one, two or three substituents, which may be the same or different, selected from hydroxy, (1-4C)alkyl and (1-4C)alkoxy;
or a pharmaceutically-acceptable salt thereof.

2. A cyclic ether derivative of the formula I as claimed in claim 1 wherein Ar¹ is phenyl or naphthyl, or a 9- or 10-membered bicyclic heterocyclic moiety containing one or two nitrogen heteroatoms and optionally containing a further heteroatom selected from nitrogen, oxygen and sulphur, and Ar¹ may optionally bear up to five substituents selected from amino, halogeno, hydroxy, cyano, oxo, thioxo, (1-4C)alkyl, (1-4C)alkoxy, (1-4C)alkylthio, (1-4C)alkylsulphinyl, (1-4C)alkylsulphonyl, (1-4C)alkylamino, di-[(1-4C)alkyl]amino, (2-4C)alkanoyl, fluoro-(1-4C)alkyl, cyano-(1-4C)alkyl, phenyl, benzoyl and phenyl-(1-4C)alkyl, and wherein said phenyl, benzoyl or phenyl-(1-4C)alkyl substituents may optionally bear a substituent selected from halogeno, (1-4C)alkyl and (1-4C)alkoxy;
wherein A¹ is a direct link to X¹ or is (1-3C)alkylene;
wherein X¹ is oxy, thio, sulphinyl, sulphonyl or imino;
wherein Ar² is phenylene which may optionally bear one or two substituents selected from halogeno, hydroxy, amino, nitro, cyano carbamoyl, ureido, (1-4C)alkyl, (1-4C)alkoxy, (1-4C)alkylamino, di-[(1-4C)alkyl]amino, fluoro-(1-4C)alkyl and (2-4C)alkanoylamino; or Ar² is pyridylene;
wherein R¹ is (1-4C)alkylthio, (1-4C)alkylsulphinyl, (1-4C)alkylsulphonyl, (1-4C)alkylamino or di-[(1-4C)alkyl]amino, or R¹ is cyano, carbamoyl, (1-4C)alkoxycarbonyl, N-(1-4C)alkylcarbamoyl, N,N-di-[(1-4C)alkyl]carbamoyl, hydroxy-(1-4C)alkyl or (1-4C)alkoxy-(1-4C)alkyl; and
wherein R² and R³ together form a group of the formula -A²-X²-A³- which, together with the carbon atom to which A² and A³ are attached, defines a ring having 5 to 7 ring atoms, wherein A² and A³, which may be the same or different, each is (1-3C)alkylene and X² is oxy, thio, sulphinyl or sulphonyl, and which ring may bear one, two or three substituents, which may be the same or different, selected from hydroxy, (1-4C)alkyl and (1-4C)alkoxy;
or a pharmaceutically-acceptable salt thereof.

3. A cyclic ether derivative of the formula I as claimed in claim 1
wherein Ar¹ is phenyl or naphth-2-yl which may optionally bear one or two substituents selected from fluoro, chloro, methyl, ethyl, isopropyl, tert-butyl, methoxy, trifluoromethyl, 2-cyanoprop-2-yl, phenyl and benzoyl and wherein said phenyl or benzoyl substituents may optionally bear a substituent selected from chloro, methyl and methoxy;
A¹ is a direct link to X¹, or is methylene;
X¹ is oxy, thio, sulphinyl or sulphonyl;
Ar² is 1,3-phenylene or 1,4-phenylene which may optionally bear one or two substituents selected from fluoro, chloro, hydroxy, amino, nitro, ureido, methoxy, dimethylamino, trifluoromethyl and acetamido; or Ar² is 3,5-pyridylene;
R¹ is methylthio, ethylthio, propylthio, methylsulphinyl, ethylsulphinyl, propylsulphinyl, methylsulphonyl, ethylsulphonyl, propylsulphonyl, methylamino, ethylamino, dimethylamino or diethylamino, or R¹ is cyano, carbamoyl, methoxycarbonyl, ethoxycarbonyl, propoxycarbonyl, N-methylcarbamoyl, N-ethylcarbamoyl, N,N-dimethylcarbamoyl, N,N-diethylcarbamoyl, hydroxymethyl, 2-hydroxyethyl, 3-hydroxypropyl, methoxymethyl, ethoxymethyl, 2-methoxyethyl, 2-ethoxyethyl, 3-methoxypropyl or 3-ethoxypropyl; and
R² and R³ together form a group of the formula -A²-X²-A³- which, together with the carbon atom to which A² and A³ are attached, defines a ring having 5 or 6 ring atoms, wherein A² is ethylene, A³ is methylene or ethylene, and X² is oxy, and which ring may bear one or two substituents selected from methyl and ethyl;
or a pharmaceutically-acceptable salt thereof.

4. A cyclic ether derivative of the formula I as claimed in claim 1
wherein Ar¹ is 2-oxoindolin-5-yl, 2,3-dioxoindolin-5-yl, 2-oxo-2,3-dihydrobenzimidazol-5-yl, 2-oxo-2,3-dihydrobenoxazol-5-yl, 2-oxo-2,3-dihydrobenzothiazol-5-yl, 2-oxo-1,2-dihydroquinolin-3-yl, 2-oxo-1,2-dihydroquinolin-6-yl, 2-oxo-1,2-dihydroquinolin-7-yl, 3-oxo-2,3-dihydro-4H-1,4-benzoxazin-7-yl or 3-oxo-2,3-dihydro-4H-1,4-benzothiazol-7-yl, which may optionally bear one, two or three substituents selected from fluoro, chloro, methyl, ethyl, 2-fluoroethyl, phenyl and benzyl;
A¹ is a direct link to X¹, or is methylene;
X¹ is oxy, thio, sulphinyl or sulphonyl;
Ar² is 1,3-phenylene or 1,4-phenylene which may optionally bear one or two substituents selected from fluoro, chloro, hydroxy, amino, nitro, ureido, methoxy, dimethylamino, trifluoromethyl and acetamido; or Ar² is 3,5-pyridylene;
R¹ is methylthio, ethylthio, propylthio, methylsulphinyl, ethylsulphinyl, propylsulphinyl, methylsulphonyl, ethylsulphonyl, propylsulphonyl, methylamino, ethylamino, dimethylamino or diethylamino, or R¹ is cyano, carbamoyl, methoxycarbonyl, ethoxycarbonyl, propoxycarbonyl, N-methylcarbamoyl, N-ethylcarbamoyl, N,N-dimethylcarbamoyl, N,N-diethylcarbamoyl, hydroxymethyl, 2-hydroxyethyl, 3-hydroxypropyl, methoxymethyl, ethoxymethyl, 2-methoxyethyl, 2-ethoxyethyl, 3-methoxypropyl or 3-ethoxypropyl; and
R² and R³ together form a group of the formula -A²-X²-A³- which, together with the carbon atom to which A² and A³ are attached, defines a ring having 5 or 6 ring atoms, wherein A² is ethylene, A³ is methylene or ethylene, and X² is oxy, and which ring may bear one or two substituents selected from methyl and ethyl;
or a pharmaceutically-acceptable salt thereof.

5. A cyclic ether derivative of the formula I as claimed in claim 1 wherein Ar¹ is naphth-2-yl which may optionally bear one or two substituents selected from fluoro, chloro, methyl, methoxy and trifluoromethyl, or Ar¹ is 2-oxo-1,2-dihydroquinolin-6-yl which may optionally bear one or two substituents selected from fluoro, chloro, methyl and ethyl;
A¹ is a direct link to X¹, or is methylene;
X¹ is oxy, thio, sulphinyl or sulphonyl;
Ar² is 1,3-phenylene which may optionally bear a substituent selected from fluoro, chloro and trifluoromethyl;
R¹ is methylthio, ethylthio, propylthio, isopropylthio, tert-butylthio, methylsulphinyl, ethylsulphinyl, propylsulphinyl, hydrogen, formyl, cyano, methoxycarbonyl, ethoxycarbonyl, propoxycarbonyl, hydroxymethyl, 1-hydroxyethyl, 2-hydroxyethyl, 2,2,2-trifluoroethylthio, acetyl, propionyl, methoxymethyl, ethoxymethyl or 2-methoxyethyl; and
R² and R³ together form a group of the formula -A²-X²-A³- which, together with the carbon atom to which each of A² and A³ is attached, defines a ring having 5 or 6 ring atoms, wherein A² is ethylene, A³ is methylene or ethylene, and X² is oxy, and which ring may bear one or two substituents selected from methyl and ethyl; or a pharmaceutically-acceptable salt thereof.

6. A cyclic ether derivative of the formula I as claimed in claim 1
wherein Ar¹ is naphth-2-yl which may optionally bear a fluoro substituent, or Ar¹ is 1-methyl-2-oxo-1,2-dihydroquinolin-6-yl;
A¹ is methylene and X¹ is oxy;
Ar² is 1,3-phenylene or 5-fluoro-1,3-phenylene;
R¹ is methylthio, ethylthio, isopropylthio, tert-butylthio, methylsulphinyl, hydrogen, formyl, cyano, methyl, ethyl, ethoxycarbonyl, hydroxymethyl, 1-hydroxyethyl, 2,2,2-trifluoroethylthio, acetyl or methoxymethyl; and
R² and R³ together form a group of the formula -A²-X²-A³- which, together with the carbon atom to which each of A² and A³ is attached, defines a ring having 6 ring atoms, wherein A² is ethylene, A³ is ethylene and X² is oxy, and which ring may bear a methyl substituent alpha to X²;
or a pharmaceutically-acceptable salt thereof.

7. A cyclic ether derivative of the formula I, or a pharmaceutically-acceptable salt thereof as defined in claim 1, selected from the group consisting of:-
4-methylthio-4-[3-(naphth-2-ylmethoxy)phenyl]tetrahydropyran and 4-ethoxycarbonyl-4-[3-(1-methyl-2-oxo-1,2-dihydroquinolin-6-ylmethoxy)phenyl]tetrahydropyran.

8. A cyclic ether derivative of the formula I, or a pharmaceutically-acceptable salt thereof as defined in claim 1, selected from the group consisting of:-
4-acetyl-4-[3-(1-methyl-2-oxo-1,2-dihydroquinolin-6-ylmethoxy)phenyl]tetrahydropyran,
4-[5-fluoro-3-(1-methyl-2-oxo-1,2-dihydroquinolin-6-ylmethoxy)phenyl]-3,4,5,6-tetrahydro-2H-pyran,
4-ethyl-4-[3-(1-methyl-2-oxo-1,2-dihydroquinolin-6-ylmethoxy)phenyl]tetrahydropyran,
(2RS,4RS)-4-ethyl-4-[5-fluoro-3-(1-methyl-2-oxo-1,2-dihydroquinolin-6-ylmethoxy)phenyl]-2-methyltetrahydropyran and
4-[5-fluoro-3-(1-methyl-2-oxo-1,2-dihydroquinolin-6-ylmethoxy)phenyl]-4-methylthiotetrahydropyran.

9. A process for the preparation of a cyclic ether derivative of the formula I, or a pharmaceutically-acceptable salt thereof, as claimed in claim 1 or claim 2 which comprises:-
(a) the coupling of a compound of the formula Ar¹-A¹-X¹-H with a compound of the formula II 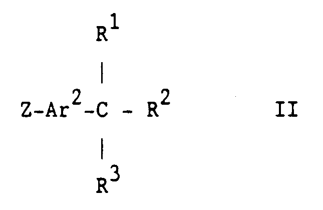 wherein Z is a displaceable group; provided that, when there is an amino, alkylamino or hydroxy group in Ar¹, Ar², R¹, R² or R³, any amino, alkylamino or hydroxy group may be protected by a conventional protecting group or alternatively any such group need not be protected, whereafter any undesired protecting group in Ar¹, Ar², R¹, R² or R³ is removed by conventional means;
(b) the coupling of a compound of the formula III 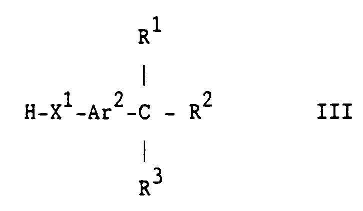 with a compound of the formula Ar¹-A¹-Z wherein Z is a displaceable group; provided that, when there is an amino, alkylamino or hydroxy group in Ar¹, Ar², R¹, R² or R³, any amino, alkylamino or hydroxy group may be protected by a conventional protecting group or alternatively any such group need not be protected, whereafter any undesired protecting group in Ar¹, Ar², R¹, R² or R³ is removed by conventional means;
(c) for the production of those compounds of the formula I wherein R¹ is (1-4C)alkylthio, the alkylation, of a compound of the formula IV 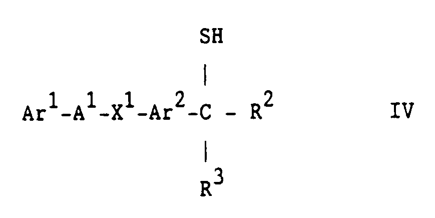 with a compound of the formula R⁴-Z, wherein Z has the meaning defined hereinbefore and R⁴ is a (1-4C)alkyl group; provided that, when there is an amino, imino, alkylamino or hydroxy group in Ar¹, Ar², X¹, R² or R³ any amino, imino, alkylamino or hydroxy group may be protected by a conventional protecting group or alternatively any such group need not be protected, whereafter any undesired protecting group in Ar¹, Ar², X¹, R² or R³ is removed by conventional means;
(d) for the production of those compounds of the formula I wherein X¹ is a sulphinyl or sulphonyl group, wherein R¹ is a (1-4C)alkylsulphinyl or (1-4C)alkylsulphonyl group, or wherein R² and R³ together form a group of the formula -A²-X²-A³- and X² is a sulphinyl or sulphonyl group, the oxidation of a compound of the formula I wherein X¹ is a thio group, wherein R¹ is a (1-4C)alkylthio group, or wherein R² and R³ together form a group of the formula -A²-X²-A³- and X² is a thio group;
(e) for the production of those compounds of the formula I wherein Ar² bears an alkanoylamino substituent, the acylation of a compound of the formula I wherein Ar² bears an amino substituent; or
(f) for the production of those compounds of the formula I wherein Ar¹ bears an alkyl or substituted alkyl substituent on an available nitrogen atom, wherein Ar² bears an alkoxy substituent or wherein R¹ is an alkoxyalkyl group, the alkylation of a compound of the formula I wherein Ar¹ bears a hydrogen atom on said available nitrogen atom, wherein Ar² bears a hydroxy substituent or wherein R¹ is a hydroxyalkyl group; and
when a pharmaceutically-acceptable salt of a novel compound of the formula I is required, it may be obtained by reaction of said compound with a suitable acid or base using a conventional procedure.

10. A pharmaceutical composition which comprises a cyclic ether derivative of the formula I, or a pharmaceutically-acceptable salt thereof, as claimed in any one of claims 1 to 8 in association with a pharmaceutically-acceptable diluent or carrier.

## Patentansprüche

1. Cyclisches Ether-Derivat mit der folgenden Formel I: 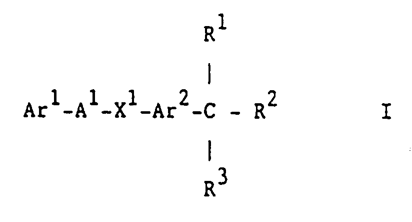 in der
Ar¹ für Phenyl oder Naphthyl oder für einen 9- oder 10-gliedrigen bicyclischen heterocyclischen Rest steht, der ein oder zwei Stickstoff-Heteroatome und gegebenenfalls ein weiteres Heteroatom enthält, das unter Stickstoff, Sauerstoff und Schwefel ausgewählt ist, wobei Ar¹ gegebenenfalls bis zu fünf Substituenten tragen kann, die unter Amino, Halogen, Hydroxy, Cyano, Oxo, Thioxo, (1-4C)Alkyl, (1-4C)Alkoxy, (1-4C)Alkylthio, (1-4C)Alkylsulfinyl, (1-4C)Alkylsulfonyl, (1-4C)Alkylamino, Di-[(1-4C)alkyl]amino, (2-4C)Alkanoyl, Fluor-(1-4C)alkyl, Cyano-(1-4C)alkyl, Phenyl, Benzoyl und Phenyl-(1-4C)alkyl ausgewählt sind, wobei die Phenyl-, Benzoyl- oder Phenyl-(1-4C)alkyl-Substituenten gegebenenfalls einen Substituenten tragen können, der unter Halogen, (1-4C)Alkyl und (1-4C)Alkoxy ausgewählt ist;
A¹ für eine Direktbindung zu X¹ oder für (1-3C)Alkylen steht;
X¹ für Oxy, Thio, Sulfinyl, Sulfonyl oder Imino steht;
Ar² für Phenylen steht, das gegebenenfalls einen oder zwei Substituenten tragen kann, die unter Halogen, Hydroxy, Amino, Nitro, Cyano, Carbamoyl, Ureido, (1-4C)Alkyl, (1-4C)Alkoxy, (1-4C)Alkylamino, Di-[(1-4C)alkyl]amino, Fluor-(1-4C)alkyl und (2-4C)Alkanoylamino ausgewählt sind; oder Ar² für Pyridylen steht;
R¹ für (1-4C)Alkylthio, (1-4C)Alkylsulfinyl, (1-4C)Alkylsulfonyl, (1-4C)Alkylamino oder Di-[(1-4C)alkyl]amino steht, oder R¹ für Wasserstoff, Formyl, Cyano, Carbamoyl, (1-4C)Alkyl, (1-4C)Alkoxycarbonyl, N-(1-4C)Alkylcarbamoyl, N,N-Di-[(1-4C)alkyl]carbamoyl, Hydroxy-(1-4C)alkyl, Fluor-(1-4C)alkylthio, (2-4C)Alkanoyl oder (1-4C)Alkoxy-(1-4C)alkyl steht; und
R² und R³ zusammen eine Gruppe mit der Formel -A²-X²-A³- bilden, die zusammen mit dem Kohlenstoffatom, an das A² und A³ gebunden sind, einen Ring mit 5 bis 7 Ringatomen definiert, wobei A² und A³, die gleich oder unterschiedlich sein können, jeweils für (1-3C)Alkylen stehen und X² für Oxy, Thio, Sulfinyl oder Sulfonyl steht, und wobei der Ring einen, zwei oder drei Substituenten, die gleich oder unterschiedlich sein können, tragen kann, die unter Hydroxy, (1-4C)Alkyl und (1-4C)Alkoxy ausgewählt sind;
oder ein pharmazeutisch geeignetes Salz davon.

2. Cyclisches Ether-Derivat mit der Formel I nach Anspruch 1, in der
Ar¹ für Phenyl oder Naphthyl oder für einen 9- oder 10-gliedrigen bicyclischen heterocyclischen Rest steht, der ein oder zwei Stickstoff-Heteroatome und gegebenenfalls ein weiteres Heteroatom enthält, das unter Stickstoff, Sauerstoff und Schwefel ausgewählt ist, wobei Ar¹ gegebenenfalls bis zu fünf Substituenten tragen kann, die unter Amino, Halogen, Hydroxy, Cyano, Oxo, Thioxo, (1-4C)Alkyl, (1-4C)Alkoxy, (1-4C)Alkylthio, (1-4C)Alkylsulfinyl, (1-4C)Alkylsulfonyl, (1-4C)Alkylamino, Di-[(1-4C)alkyl]amino, (2-4C)Alkanoyl, Fluor-(1-4C)alkyl, Cyano-(1-4C)alkyl, Phenyl, Benzoyl und Phenyl-(1-4C)alkyl ausgewählt sind, wobei die Phenyl-, Benzoyl- oder Phenyl-(1-4C)alkyl-Substituenten gegebenenfalls einen Substituenten tragen können, der unter Halogen, (1-4C)Alkyl und (1-4C)Alkoxy ausgewählt ist;
A¹ für eine Direktbindung zu X¹ oder für (1-3C)Alkylen steht;
X¹ für Oxy, Thio, Sulfinyl, Sulfonyl oder Imino steht;
Ar² für Phenylen steht, das gegebenenfalls einen oder zwei Substituenten tragen kann, die unter Halogen, Hydroxy, Amino, Nitro, Cyano, Carbamoyl, Ureido, (1-4C)Alkyl, (1-4C)Alkoxy, (1-4C)Alkylamino, Di-[(1-4C)alkyl]amino, Fluor-(1-4C)alkyl und (2-4C)Alkanoylamino ausgewählt sind; oder
Ar² für Pyridylen steht;
R¹ für (1-4C)Alkylthio, (1-4C)Alkylsulfinyl, (1-4C)Alkylsulfonyl, (1-4C)Alkylamino oder Di-[(1-4C)alkyl]amino steht, oder
R¹ für Cyano, Carbamoyl, (1-4C)Alkoxycarbonyl, N-(1-4C)Alkylcarbamoyl, N,N-Di-[(1-4C)alkyl]carbamoyl, Hydroxy-(1-4C)alkyl oder (1-4C)Alkoxy-(1-4C)alkyl steht; und
R² und R³ zusammen eine Gruppe mit der Formel -A²-X²-A³- bilden, die zusammen mit dem Kohlenstoffatom, an das A² und A³ gebunden sind, einen Ring mit 5 bis 7 Ringatomen definiert, wobei A² und A³, die gleich oder unterschiedlich sein können, jeweils für (1-3C)Alkylen stehen und X² für Oxy, Thio, Sulfinyl oder Sulfonyl steht, wobei der Ring einen, zwei oder drei Substituenten, die gleich oder unterschiedlich sein können, tragen kann, die unter Hydroxy, (1-4C)Alkyl und (1-4C)Alkoxy ausgewählt sind;
oder ein pharmazeutisch geeignetes Salz davon.

3. Cyclisches Ether-Derivat mit der Formel I nach Anspruch 1, in der
Ar¹ für Phenyl oder Napth-2-yl steht, das gegebenenfalls einen oder zwei Substituenten tragen kann, die unter Fluor, Chlor, Methyl, Ethyl, Isopropyl, tert-Butyl, Methoxy, Trifluormethyl, 2-Cyanoprop-2-yl, Phenyl und Benzoyl ausgewählt sind, wobei die Phenyl- oder Benzoyl-Substituenten gegebenenfalls einen Substituenten tragen können, der unter Chlor, Methyl und Methoxy ausgewählt ist;
A¹ für eine Direktbindung zu X¹ oder für Methylen steht;
X¹ für Oxy, Thio, Sulfinyl oder Sulfonyl steht;
Ar² für 1,3-Phenylen oder 1,4-Phenylen steht, das gegebenenfalls einen oder zwei Substituenten tragen kann, die unter Fluor, Chlor, Hydroxy, Amino, Nitro, Ureido, Methoxy, Dimethylamino, Trifluormethyl und Acetamido ausgewählt sind; oder
Ar² für 3,5-Pyridylen steht;
R¹ für Methylthio, Ethylthio, Propylthio, Methylsulfinyl, Ethylsulfinyl, Propylsulfinyl, Methylsulfonyl, Ethylsulfonyl, Propylsulfonyl, Methylamino, Ethylamino, Dimethylamino oder Diethylamino steht, oder
R¹ für Cyano, Carbamoyl, Methoxycarbonyl, Ethoxycarbonyl, Propoxycarbonyl, N-Methylcarbamoyl, N-Ethylcarbamoyl, N,N-Dimethylcarbamoyl, N,N-Diethylcarbamoyl, Hydroxymethyl, 2-Hydroxyethyl, 3-Hydroxypropyl, Methoxymethyl, Ethoxymethyl, 2-Methoxyethyl, 2-Ethoxyethyl, 3-Methoxypropyl oder 3-Ethoxypropyl steht; und
R² und R³ zusammen eine Gruppe mit der Formel -A²-X²-A³- bilden, die zusammen mit dem Kohlenstoffatom, an das A² und A³ gebunden sind, einen Ring mit 5 oder 6 Ringatomen definiert, wobei A² für Ethylen steht, A³ für Methylen oder Ethylen steht und X² für Oxy steht, und wobei der Ring einen oder zwei Substituenten tragen kann, die unter Methyl und Ethyl ausgewählt sind;
oder ein pharmazeutisch geeignetes Salz davon.

4. Cyclisches Ether-Derivat mit der Formel I nach Anspruch 1, in der
Ar¹ für 2-Oxoindolin-5-yl, 2,3-Dioxoindolin-5-yl, 2-Oxo-2,3-dihydrobenzimidazol-5-yl, 2-Oxo-2,3-dihydrobenzoxazol-5-yl, 2-Oxo-2,3-dihydrobenzothiazol-5-yl, 2-Oxo-1,2-dihydrochinolin-3-yl, 2-Oxo-1,2-dihydrochinolin-6-yl, 2-Oxo-1,2-dihydrochinolin-7-yl, 3-Oxo-2,3-dihydro-4H-1,4-benzoxazin-7-yl oder 3-Oxo-2,3-dihydro-4H-1,4-benzothiazol-7-yl steht, das gegebenenfalls einen, zwei oder drei Substituenten tragen kann, die unter Fluor, Chlor, Methyl, Ethyl, 2-Fluorethyl, Phenyl und Benzyl ausgewählt sind;
A¹ für eine Direktbindung zu X¹ oder für Methylen steht;
X¹ für Oxy, Thio, Sulfinyl oder Sulfonyl steht;
Ar² für 1,3-Phenylen oder 1,4-Phenylen steht, das gegebenenfalls einen oder zwei Substituenten tragen kann, die unter Fluor, Chlor, Hydroxy, Amino, Nitro, Ureido, Methoxy, Dimethylamino, Trifluormethyl und Acetamido ausgewählt sind; oder
Ar² für 3,5-Pyridylen steht;
R¹ für Methylthio, Ethylthio, Propylthio, Methylsulfinyl, Ethylsulfinyl, Propylsulfinyl, Methylsulfonyl, Ethylsulfonyl, Propylsulfonyl, Methylamino, Ethylamino, Dimethylamino oder Diethylamino steht, oder
R¹ für Cyano, Carbamoyl, Methoxycarbonyl, Ethoxycarbonyl, Propoxycarbonyl, N-Methylcarbamoyl, N-Ethylcarbamoyl, N,N-Dimethylcarbamoyl, N,N-Diethylcarbamoyl, Hydroxymethyl, 2-Hydroxyethyl, 3-Hydroxypropyl, Methoxymethyl, Ethoxymethyl, 2-Methoxyethyl, 2-Ethoxyethyl, 3-Methoxypropyl oder 3-Ethoxypropyl steht; und
R² und R³ zusammen eine Gruppe mit der Formel -A²-X²-A³- bilden, die zusammen mit dem Kohlenstoffatom, an das A² und A³ gebunden sind, einen Ring mit 5 oder 6 Ringatomen definiert, wobei A² für Ethylen steht, A³ für Methylen oder Ethylen steht und X² für Oxy steht, und wobei der Ring einen oder zwei Substituenten tragen kann, die unter Methyl und Ethyl ausgewählt sind;
oder ein pharmazeutisch geeignetes Salz davon.

5. Cyclisches Ether-Derivat mit der Formel I nach Anspruch 1, in der
Ar¹ für Naphth-2-yl steht, das gegebenenfalls einen oder zwei Substituenten tragen kann, die unter Fluor, Chlor, Methyl, Methoxy und Trifluormethyl ausgewählt sind, oder
Ar¹ für 2-Oxo-1,2-dihydrochinolin-6-yl steht, das gegebenenfalls einen oder zwei Substituenten tragen kann, die unter Fluor, Chlor, Methyl und Ethyl ausgewählt sind;
A¹ für eine Direktbindung zu X¹ oder für Methylen steht;
X¹ für Oxy, Thio, Sulfinyl oder Sulfonyl steht;
Ar² für 1,3-Phenylen steht, das gegebenenfalls einen Substituenten tragen kann, der unter Fluor, Chlor und Trifluormethyl ausgewählt ist;
R¹ für Methylthio, Ethylthio, Propylthio, Isopropylthio, tert-Butylthio, Methylsulfinyl, Ethylsulfinyl, Propylsulfinyl, Wasserstoff, Formyl, Cyano, Methoxycarbonyl, Ethoxycarbonyl, Propoxycarbonyl, Hydroxymethyl, 1-Hydroxyethyl, 2-Hydroxyethyl, 2,2,2-Trifluorethylthio, Acetyl, Propionyl, Methoxymethyl, Ethoxymethyl oder 2-Methoxyethyl steht; und
R² und R³ zusammen eine Gruppe mit der Formel -A²-X²-A³- bilden, die zusammen mit dem Kohlenstoffatom, an das A² und A³ jeweils gebunden sind, einen Ring mit 5 oder 6 Ringatomen definiert, wobei A² für Ethylen steht, A³ für Methylen oder Ethylen steht und X² für Oxy steht, und wobei der Ring einen oder zwei Substituenten tragen kann, die unter Methyl und Ethyl ausgewählt sind;
oder ein pharmazeutisch geeignetes Salz davon.

6. Cyclisches Ether-Derivat mit der Formel I nach Anspruch 1, in der
Ar¹ für Naphth-2-yl steht, das gegebenenfalls einen Fluor-Substituenten tragen kann, oder
Ar¹ für 1-Methyl-2-oxo-1,2-dihydrochinolin-6-yl steht;
A¹ für Methylen steht und X¹ für Oxy steht;
Ar² für 1,3-Phenylen oder 5-Fluor-1,3-phenylen steht;
R¹ für Methylthio, Ethylthio, Isopropylthio, tert-Butylthio, Methylsulfinyl, Wasserstoff, Formyl, Cyano, Methyl, Ethyl, Ethoxycarbonyl, Hydroxymethyl, 1-Hydroxyethyl, 2,2,2-Trifluorethylthio, Acetyl oder Methoxymethyl steht; und
R² und R³ zusammen eine Gruppe mit der Formel -A²-X²-A³- bilden, die zusammen mit dem Kohlenstoffatom, an das A² und A³ jeweils gebunden sind, einen Ring mit 6 Ringatomen definiert, wobei A² für Ethylen steht, A³ für Ethylen steht und X² für Oxy steht, und wobei der Ring einen Methyl-Substituenten alpha zu X² tragen kann;
oder ein pharmazeutisch geeignetes Salz davon.

7. Cyclisches Ether-Derivat mit der Formel I, oder ein pharmazeutisch geeignetes Salz davon, nach Anspruch 1, das aus der aus folgendem bestehenden Gruppe ausgewählt ist:-
4-Methylthio-4-[3-(napht-2-ylmethoxy)phenyl]tetrahydropyran und
4-Ethoxycarbonyl-4-[3-(1-methyl-2-oxo-1,2-dihydrochinolin-6-ylmethoxy)phenyl]tetrahydropyran.

8. Cyclisches Ether-Derivat mit der Formel I, oder ein pharmazeutisch geeignetes Salz davon, nach Anspruch 1, das aus der aus folgendem bestehenden Gruppe ausgewählt ist:-
4-Acetyl-4-[3-(1-methyl-2-oxo-1,2-dihydrochinolin-6-ylmethoxy)phenyl]tetrahydropyran,
4-[5-Fluor-3-(1-methyl-2-oxo-1,2-dihydrochinolin-6-ylmethoxy)phenyl]-3,4,5,6-tetrahydro-2H-pyran,
4-Ethyl-4-[3-(1-methyl-2-oxo-1,2-dihydrochinolin-6-ylmethoxy)phenyl]tetrahydropyran,
(2RS,4RS)-4-Ethyl-4-[5-fluor-3-(1-methyl-2-oxo-1,2-dihydrochinolin-6-ylmethoxy)phenyl]-2-methyltetrahydropyran und
4-[5-Fluor-3-(1-methyl-2-oxo-1,2-dihydrochinolin-6-ylmethoxy)phenyl]-4-methylthiotetrahydropyran.

9. Verfahren zur Herstellung eines cyclischen Ether-Derivats mit der Formel I, oder eines pharmazeutisch geeigneten Salzes davon, nach Anspruch 1 oder Anspruch 2, bei dem:-
(a) eine Verbindung mit der Formel Ar¹-A¹-X¹-H mit einer Verbindung mit der folgenden Formel II: 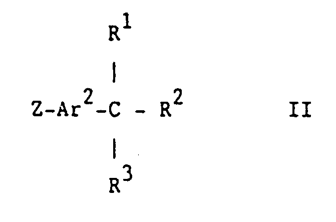 in der Z für eine austauschbare Gruppe steht, gekuppelt wird, mit der Maßgabe, daß, wenn eine Amino-, Alkylamino- oder Hydroxy-Gruppe in Ar¹, Ar², R¹, R² oder R³ vorhanden ist, jede Amino-, Alkylamino- oder Hydroxy-Gruppe mit einer üblichen Schutzgruppe geschützt werden kann oder alternativ keine derartige Gruppe geschützt werden muß, woraufhin jede unerwünschte Schutzgruppe in Ar¹, Ar², R¹, R² oder R³ mit üblichen Mitteln entfernt wird.;
(b) eine Verbindung mit der folgenden Formel III: 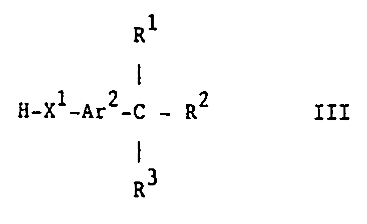 mit einer Verbindung mit der Formel Ar¹-A¹-Z, in der Z für eine austauscbbare Gruppe steht, gekuppelt wird, mit der Maßgabe, daß, wenn eine Amino-, Alkylamino- oder Hydroxy-Gruppe in Ar¹, Ar², R¹, R² oder R³ vorhanden ist, jede Amino-, Alkylamino- oder Hydroxy-Gruppe mit einer üblichen Schutzgruppe geschützt werden kann oder alternativ keine derartige Gruppe geschützt werden muß, woraufhin jede unerwünschte Schutzgruppe in Ar¹, Ar², R¹, R² oder R³ mit üblichen Mitteln entfernt wird;
(c) zur Herstellung derjenigen Verbindungen mit der Formel I, bei denen R¹ für (1-4C)Alkylthio steht, eine Verbindung mit der folgenden Formel IV 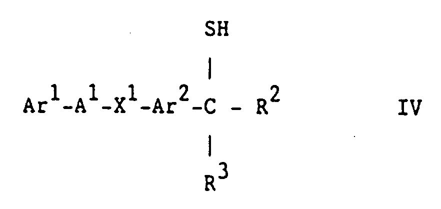 mit einer Verbindung mit der Formel R⁴-Z, in der Z die oben definierte Bedeutung hat und R⁴ für eine (1-4C)Alkyl-Gruppe steht, alkyliert wird, mit der Maßgabe, daß, wenn eine Amino-, Imino-, Alkylamino- oder Hydroxy-Gruppe in Ar¹, Ar², X¹, R² oder R³ vorhanden ist, jede Amino-, Imino-, Alkylamino- oder Hydroxy-Gruppe mit einer üblichen Schutzgruppe geschützt sein kann oder alternativ keine derartige Gruppe geschützt werden muß, woraufhin jede unerwünschte Schutzgruppe in Ar¹, Ar², X¹, R² oder R³ mit üblichen Mitteln entfernt wird;
(d) zur Herstellung derjenigen Verbindungen mit der Formel I, bei denen X¹ für eine Sulfinyl- oder Sulfonyl-Gruppe steht, bei denen R¹ für eine (1-4C)Alkylsulfinyl- oder (1-4C)Alkylsulfonyl-Gruppe steht, oder bei denen R² und R³ zusammen eine Gruppe mit der Formel -A²-X²-A³- bilden und X² für eine Sulfinyl- oder Sulfonyl-Gruppe steht, eine Verbindung mit der Formel I, bei der X¹ für eine Thio-Gruppe steht, bei der R¹ für eine (1-4C)Alkylthio-Gruppe steht, oder bei der R² und R³ zusammen eine Gruppe mit der Formel -A²-X²-A³- bilden und X² für eine Thio-Gruppe steht, oxidiert wird;
(e) zur Herstellung derjenigen Verbindungen mit der Formel I, bei denen Ar² einen Alkanoylamino-Substituenten trägt, eine Verbindung mit der Formel I, bei der Ar² einen Amino-Substituenten trägt, acyliert wird; oder
(f) zur Herstellung derjenigen Verbindungen mit der Formel I, bei denen Ar¹ einen Alkyl- oder substituierten Alkyl-Substituenten an einem verfügbaren Stickstoffatom trägt, bei denen Ar² einen Alkoxy-Substituenten trägt, oder bei denen R¹ für eine Alkoxyalkyl-Gruppe steht, eine Verbindung mit der Formel I, bei der Ar¹ ein Wasserstoffatom an dem verfügbaren Stickstoffatom trägt, bei der Ar² einen Hydroxy-Substituenten trägt, oder bei der R¹ für eine Hydroxyalkyl-Gruppe steht, alkyliert wird; und
wenn ein pharmazeutisch geeignetes Salz einer neuen Verbindung mit der Formel I benötigt wird, dies durch Umsetzung der Verbindung mit einer geeigneten Säure oder Base unter Anwendung eines üblichen Verfahrens erhältlich ist.

10. Pharmazeutische Zusammensetzung, die ein cyclisches Ether-Derivat mit der Formel I, oder ein pharmazeutisch geeignetes Salz davon, nach einem der Ansprüche 1 bis 8 in Verbindung mit einem pharmazeutisch geeigneten Streckmittel oder Trägermittel enthält.

## Revendications

1. Dérivé d'éther cyclique de formule I 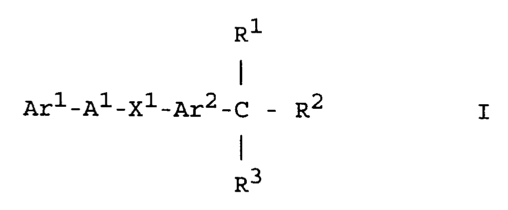 dans laquelle Ar¹ représente un groupe phényle ou naphtyle, ou un groupement hétérocyclique, bicyclique, nona- ou décagonal contenant un ou deux hétéroatomes d'azote et contenant facultativement un hétéroatome supplémentaire choisi entre l'azote, l'oxygène et le soufre, Ar¹ pouvant porter facultativement jusqu'à cinq substituants choisis entre des substituants amino, halogéno, hydroxy, cyano, oxo, thioxo, alkyle en C₁ à C₄, alkoxy en C₁ à C₄, alkylthio en C₁ à C₄, alkylsulfinyle en C₁ à C₄, alkylsulfonyle en C₁ à C₄, alkylamino en C₁ à C₄, di-(alkyle en C₁ à C₄)amino, alcanoyle en C₂ à C₄, fluoralkyle en C₁ à C₄, cyano-(alkyle en C₁ à C₄), phényle, benzoyle et phényl-(alkyle en C₁ à C₄), et lesdits substituants phényle, benzoyle ou phényl-(alkyle en C₁ à C₄) pouvant porter facultativement un substituant choisi entre des substituants halogéno, alkyle en C₁ à C₄ et alkoxy en C₁ à C₄ ;
A¹ représente une liaison directe à X¹ ou un groupe alkylène en C₁ à C₃ ;
X¹ représente un groupe oxy, thio, sulfinyle, sulfonyle ou imino ;
Ar² représente un groupe phénylène qui peut porter facultativement un ou deux substituants choisis entre des substituants halogéno, hydroxy, amino, nitro, cyano, carbamoyle, uréido, alkyle en C₁ à C₄, alkoxy en C₁ à C₄, alkylamino en C₁ à C₄, di-(alkyle en C₁ à C₄)amino, fluoralkyle en C₁ à C₄ et alcanoylamino en C₂ à C₄ ; ou Ar² représente un groupe pyridylène ;
R¹ représente un groupe alkylthio en C₁ à C₄, alkylsulfinyle en C₁ à C₄, alkylsulfonyle en C₁ à C₄, alkylamino en C₁ à C₄ ou di-(alkyle en C₁ à C₄)amino, ou R¹ représente l'hydrogène, un groupe formyle, cyano, carbamoyle, alkyle en C₁ à C₄, (alkoxy en C₁ à C₄)-carbonyle, N-(alkyle en C₁ à C₄)-carbamoyle, N,N-di-(alkyle en C₁ à C₄)-carbamoyle, hydroxyalkyle en C₁ à C₄, fluoralkylthio en C₁ à C₄, alcanoyle en C₂ à C₄ ou (alkoxy en C₁ à C₄)-(alkyle en C₁ à C₄) ; et
R² et R³ forment conjointement un groupe de formule -A²-X²-A³- qui, conjointement avec l'atome de carbone auquel A² et A³ sont fixés, définit un noyau ayant 5 à 7 atomes dans le cycle, A² et A³, qui peuvent être identiques ou différents, représentant chacun un groupe alkylène en C₁ à C₃ et X² représentant un groupe oxy, thio, sulfinyle ou sulfonyle, noyau qui peut porter un, deux ou trois substituants qui peuvent être identiques ou différents et sont choisis entre des groupes hydroxy, alkyle en C₁ à C₄ et alkoxy en C₁ à C₄ ;
ou un de ses sels pharmaceutiquement acceptables.

2. Dérivé d'éther cyclique de formule I suivant la revendication 1, dans lequel Ar¹ représente un groupe phényle ou naphtyle, ou un groupement hétérocyclique, bicyclique, nona- ou décagonal contenant un ou deux hétéroatomes d'azote et contenant facultativement un hétéroatome supplémentaire choisi entre l'azote, l'oxygène et le soufre, Ar¹ pouvant porter facultativement jusqu'à cinq substituants choisis entre des substituants amino, halogéno, hydroxy, cyano, oxo, thioxo, alkyle en C₁ à C₄, alkoxy en C₁ à C₄, alkylthio en C₁ à C₄, alkylsulfinyle en C₁ à C₄, alkylsulfonyle en C₁ à C₄, alkylamino en C₁ à C₄, di-(alkyle en C₁ à C₄)amino, alcanoyle en C₂ à C₄, fluoralkyle en C₁ à C₄, cyano-(alkyle en C₁ à C₄), phényle, benzoyle et phényl-(alkyle en C₁ à C₄), lesdits substituants phényle, benzoyle et phényl-(alkyle en C₁ à C₄) pouvant porter facultativement un substituant choisi entre des groupes halogéno, alkyle en C₁ à C₄ et alkoxy en C₁ à C₄ ;
A¹ représente une liaison directe à X¹ ou un groupe alkylène en C₁ à C₃ ;
X¹ représente un groupe oxy, thio, sulfinyle, sulfonyle ou imino ;
Ar² représente un groupe phénylène qui peut porter facultativement un ou deux substituants choisis entre des groupes halogéno, hydroxy, amino, nitro, cyano, carbamoyle, uréido, alkyle en C₁ à C₄, alkoxy en C₁ à C₄, alkylamino en C₁ à C₄, di-(alkyle en C₁ à C₄)amino, fluoralkyle en C₁ à C₄ et alcanoylamino en C₂ à C₄ ; ou Ar² représente un groupe pyridylène ;
R¹ représente un groupe alkylthio en C₁ à C₄, alkylsulfinyle en C₁ à C₄, alkylsulfonyle en C₁ à C₄, alkylamino en C₁ à C₄ ou di-(alkyle en C₁ à C₄)amino, ou R¹ représente un groupe cyano, carbamoyle, (alkoxy en C₁ à C₄)-carbonyle, N-(alkyle en C₁ à C₄)-carbamoyle, N,N-di-(alkyle en C₁ à C₄)-carbamoyle, hydroxyalkyle en C₁ à C₄ ou (alkoxy en C₁ à C₄)-(alkyle en C₁ à C₄) ; et
R² et R³ forment conjointement un groupe de formule -A²-X²-A³- qui, conjointement avec l'atome de carbone auquel A² et A³ sont fixés, définit un noyau ayant 5 à 7 atomes dans le cycle, A² et A³, qui peuvent être identiques ou différents, représentant chacun un groupe alkylène en C₁ à C₃ et X² représentant un groupe oxy, thio, sulfinyle ou sulfonyle, noyau qui peut porter un, deux ou trois substituants qui peuvent être identiques ou différents et qui sont choisis entre des groupes hydroxy, alkyle en C₁ à C₄ et alkoxy en C₁ à C₄ ;
ou un de ses sels pharmaceutiquement acceptables.

3. Dérivé d'éther cyclique de formule I suivant la revendication 1,
dans lequel Ar¹ représente un groupe Phényle ou naphtyl-2-yle qui peut porter facultativement un ou deux substituants choisis entre des groupes fluoro, chloro, méthyle, éthyle, isopropyle, tertio-butyle, méthoxy, trifluorométhyle, 2-cyanoprop-2-yle, phényle et benzoyle, lesdits substituants phényle et benzoyle pouvant porter facultativement un substituant choisi entre les groupes chloro, méthyle et méthoxy ;
A¹ représente une liaison directe à X¹ ou un groupe méthylène ;
X¹ représente un groupe oxo, thio, sulfinyle ou sulfonyle ;
Ar² représente un groupe 1,3-phénylène ou 1,4-phénylène qui peut porter facultativement un ou deux substituants choisis entre les groupes fluoro, chloro, hydroxy, amino, nitro, uréido, méthoxy, diméthylamino, trifluorométhyle et acétamido ; ou bien Ar² représente un groupe 3,5-pyridylène ;
R¹ représente un groupe méthylthio, éthylthio, propylthio, méthylsulfinyle, éthylsulfinyle, propylsulfinyle, méthylsulfonyle, éthylsulfonyle, propylsulfonyle, méthylamino, éthylamino, diméthylamino ou diéthylamino, ou bien R¹ représente un groupe cyano, carbamoyle, méthoxycarbonyle, éthoxycarbonyle, propoxycarbonyle, N-méthylcarbamoyle, N-éthylcarbamoyle, N,N-diméthylcarbamoyle, N,N-diéthylcarbamoyle, hydroxyméthyle, 2-hydroxyéthyle, 3-hydroxypropyle, méthoxyméthyle, éthoxyméthyle, 2-méthoxyéthyle, 2-éthoxyéthyle, 3-méthoxypropyle ou 3-éthoxypropyle ; et R² et R³ forment conjointement un groupe de formule -A²-X²-A³- qui, conjointement avec l'atome de carbone auquel A² et A³ sont fixés, définit un noyau ayant 5 ou 6 atomes dans le cycle, A² représentant un groupe éthylène, A³ représentant un groupe méthylène ou éthylène et X² représentant un groupe oxy, noyau qui peut porter un ou deux substituants choisis entre les groupes méthyle et éthyle ;
ou un de ses sels pharmaceutiquement acceptables.

4. Dérivé d'éther cyclique de formule I suivant la revendication 1,
dans lequel Ar¹ représente un groupe 2-oxo-indoline-5-yle, 2,3-dioxoindoline-5-yle, 2-oxo-2,3-dihydrobenzimidazole-5-yle, 2-oxo-2,3-dihydrobenzoxazole-5-yle, 2-oxo-2,3-dihydrobenzothiazole-5-yle, 2-oxo-1,2-dihydroquinoléine-3-yle, 2-oxo-1,2-dihydroquinoléine-6-yle, 2-oxo-1,2-dihydroquinoléine-7-yle, 3-oxo-2,3-dihydro-4H-1,4-benzoxazine-7-yle ou 3-oxo-2,3-dihydro-4H-1,4-benzothiazole-7-yle, qui peut porter facultativement un, deux ou trois substituants choisis entre les groupes fluoro, chloro, méthyle, éthyle, 2-fluoréthyle, phényle et benzyle ;
A¹ représente une liaison directe à X¹ ou un groupe méthylène ;
X¹ représente un groupe oxy, thio, sulfinyle ou sulfonyle ;
Ar² représente un groupe 1,3-phénylène ou 1,4-phénylène qui peut porter facultativement un ou deux substituants choisis entre les groupes fluoro, chloro, hydroxy, amino, nitro, uréido, méthoxy, diméthylamino, trifluorométhyle et acétamido ; ou bien Ar² représente un groupe 3,5-pyridylène ;
R¹ représente un groupe méthylthio, éthylthio, propylthio, méthylsulfinyle, éthylsulfinyle, propylsulfinyle, méthylsulfonyle, éthylsulfonyle, propylsulfonyle, méthylamino, éthylamino, diméthylamino ou diéthylamino, ou bien R¹ représente un groupe cyano, carbamoyle, méthoxycarbonyle, éthoxycarbonyle, propoxycarbonyle, N-méthylcarbamoyle, N-éthylcarbamoyle, N,N-diméthylcarbamoyle, N,N-diéthylcarbamoyle, hydroxyméthyle, 2-hydroxyéthyle, 3-hydroxypropyle, méthoxyméthyle, éthoxyméthyle, 2-méthoxyéthyle, 2-éthoxyéthyle, 3-méthoxypropyle ou 3-éthoxypropyle ; et
R² et R³ forment conjointement un groupe de formule -A²-X²-A³- qui, conjointement avec l'atome de carbone auquel A² et A³ sont fixés, définit un noyau ayant 5 ou 6 atomes dans le cycle, A² représentant un groupe éthylène, A³ représentant un groupe méthylène ou éthylène et X² représentant un groupe oxy, noyau qui peut porter un ou deux substituants choisis entre les groupes méthyle et éthyle ;
ou un de ses sels pharmaceutiquement acceptables.

5. Dérivé d'éther cyclique de formule I suivant la revendication 1, dans lequel Ar¹ représente un groupe napht-2-yle qui peut porter facultativement un ou deux substituants choisis entre les groupes fluoro, chloro, méthyle, méthoxy et trifluorométhyle, ou bien Ar¹ représente un groupe 2-oxo-1,2-dihydroquinoléine-6-yle qui peut porter facultativement un ou deux substituants choisis entre les groupes fluoro, chloro, méthyle et éthyle ;
A¹ représente une liaison directe à X¹ ou un groupe méthylène ;
X¹ représente un groupe oxy, thio, sulfinyle ou sulfonyle ;
Ar² représente un groupe 1,3-phénylène qui peut porter facultativement un substituant choisi entre les groupes fluoro, chloro et trifluorométhyle ;
R¹ représente un groupe méthylthio, éthylthio, propylthio, isopropylthio, tertio-butylthio, méthylsulfinyle, éthylsulfinyle, propylsulfinyle, hydrogène, formyle, cyano, méthoxycarbonyle, éthoxycarbonyle, propoxycarbonyle, hydroxyméthyle, 1-hydroxyéthyle, 2-hydroxyéthyle, 2,2,2-trifluoréthylthio, acétyle, propionyle, méthoxyméthyle, éthoxyméthyle ou 2-méthoxyéthyle ; et
R² et R³ forment conjointement un groupe de formule -A²-X²-A³- qui, conjointement avec l'atome de carbone auquel A² et A³ sont chacun fixés, définit un noyau ayant 5 ou 6 atomes dans le cycle, et X² représente un groupe oxy, noyau qui peut porter un ou deux substituants choisis entre les groupes méthyle et éthyle ;
ou un de ses sels pharmaceutiquement acceptables.

6. Dérivé d'éther cyclique de formule I suivant la revendication 1,
dans lequel Ar¹ représente un grouope napht-2-yle qui peut porter facultativement un substituant fluoro, ou bien Ar¹ représente un groupe 1-méthyl-2-oxo-1,2-dihydroquinoléine-6-yle ;
A¹ représente un groupe méthylène et X¹ représente un groupe oxy ;
Ar² représente un groupe 1,3-phénylène ou 5-fluoro-1,3-phénylène ;
R¹ représente un groupe méthylthio, éthylthio, isopropylthio, tertio-butylthio, méthylsulfinyle, hydrogène, formyle, cyano, méthyle, éthyle, éthoxycarbonyle, hydroxyméthyle, 1-hydroxyéthyle, 2,2,2-trifluoréthylthio, acétyle ou méthoxyméthyle ; et
R² et R³ forment conjointement un groupe de formule -A²-X²-A³- qui, conjointement avec l'atome de carbone auquel A² et A³ sont chacun fixés, définit un noyau ayant 6 atomes dans le cycle, A² représentant un groupe éthylène, A³ représentant un groupe éthylène et X² représentant un groupe oxy, noyau qui peut porter un substituant méthyle en position alpha par rapport à X² ;
ou un de ses sels pharmaceutiquement acceptables.

7. Dérivé d'éther cyclique de formule I, ou un de ses sels pharmaceutiquement acceptables suivant la revendication 1, choisi dans le groupe consistant en :
le 4-méthylthio-4-[3-(napht-2-ylméthoxy)phényl]tétrahydropyranne et le 4-éthoxycarbonyl-4-[3-(1-méthyl-2-oxo-1,2-dihydroquinoléine-6-ylméthoxy)phényl]tétrahydropyranne.

8. Dérivé d'éther cyclique de formule I, ou un de ses sels pharmaceutiquement acceptables suivant la revendication 1, choisi dans le groupe consistant en :
le 4-acétyl-4-[3-(1-méthyl-2-oxo-1,2-dihydroquinoléine-6-ylméthoxy)phényl]tétrahydropyranne,
le 4-[5-fluoro-3-(1-méthyl-2-oxo-1,2-dihydroquinoléine-6-ylméthoxy)phényl]-3,4,5,6-tétrahydro-2H-pyranne,
le 4-éthyl-4-[3-(1-méthyl-2-oxo-1,2-dihydroquinoléine-6-ylméthoxy)phényl]tétrahydropyranne,
le (2RS,4RS)-4-éthyl-4-[5-fluoro-3-(1-méthyl-2-oxo-1,2-dihydroquinoléine-6-ylméthoxy)phényl]-2-méthyltétrahydropyranne, et
le 4-[5-fluoro-3-(1-méthyl-2-oxo-1,2-dihydroquinoléine-6-ylméthoxy)phényl]-4-méthylthiotétrahydropyranne.

9. Procédé de préparation d'un dérivé d'éther cyclique de formule I, ou d'un de ses sels pharmaceutiquement acceptables, suivant la revendication 1 ou la revendication 2, qui comprend :
(a) le couplage d'un composé de formule Ar¹-A¹-X¹-H avec un composé de formule II 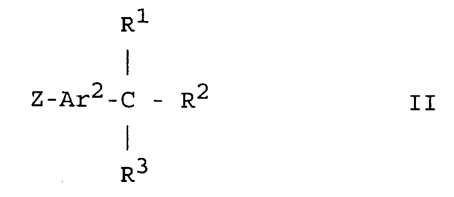 dans laquelle Z représente un groupe déplaçable ; sous réserve que, lorsqu'il existe un groupe amino, alkylamino ou hydroxy dans Ar¹, Ar², R¹, R² ou R³, n'importe quel groupe amino, alkylamino ou hydroxy puisse être protégé avec un groupe protecteur classique ou, en variante, n'importe quel groupe de ce type ne nécessite pas une protection, tout groupe protecteur non désiré dans Ar¹, Ar², R¹, R² ou R³ étant ensuite éliminé par un moyen classique ;
(b) le couplage d'un composé de formule III 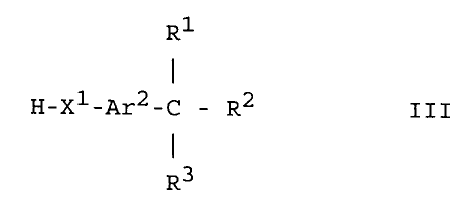 avec un composé de formule Ar¹-A¹-Z dans laquelle Z représente un groupe déplaçable ; sous réserve que, lorsqu'il existe un groupe amino, alkylamino ou hydroxy dans Ar¹, Ar², R¹, R² ou R³, n'importe quel groupe amino, alkylamino ou hydroxy puisse être protégé avec un groupe protecteur classique ou, en variante, n'importe quel groupe de ce type ne nécessite pas une protection, tout groupe protecteur non désiré dans Ar¹, Ar², R¹, R² ou R³ étant ensuite éliminé par un moyen classique ;
(c) pour la production des composés de formule I dans laquelle R¹ représente un groupe alkylthio en C₁ à C₄, l'alkylation d'un composé de formule IV 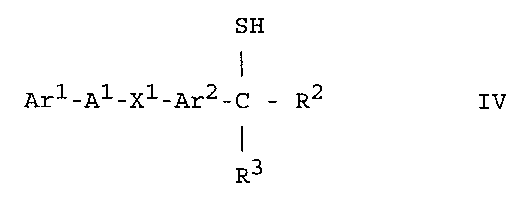 avec un composé de formule R⁴-Z, dans laquelle Z répond à la définition précitée et R⁴ représente un groupe alkyle en C₁ à C₄ ; sous réserve que, lorsqu'il existe un groupe amino, imino, alkylamino ou hydroxy dans Ar¹, Ar², R¹, R² ou R³, n'importe quel groupe amino, imino, alkylamino ou hydroxy puisse être protégé avec un groupe protecteur classique ou, en variante, n'importe quel groupe de ce type ne nécessite pas une protection, tout groupe protecteur non désiré dans Ar¹, Ar², R¹, R² ou R³ étant ensuite éliminé par un moyen classique ;
(d) pour la production des composés de formule I dans laquelle X¹ représente un groupe sulfinyle ou sulfonyle, dans laquelle R¹ représente un groupe alkylsulfinyle en C₁ à C₄ ou alkylsulfonyle en C₁ à C₄, ou dans laquelle R² et R³ forment conjointement un groupe de formule -A²-X²-A³- et X² représente un groupe sulfinyle ou sulfonyle, l'oxydation d'un composé de formule I dans laquelle X¹ représente un groupe thio, dans laquelle R¹ représente un groupe alkylthio en C₁ à C₄, ou dans laquelle R² et R³ forment conjointement un groupe de formule -A²-X²-A³- et X² représente un groupe thio ;
(e) pour la production des composés de formule I, dans laquelle Ar² porte un substituant alcanoylamino, l'acylation d'un composé de formule I dans laquelle Ar² porte un substituant amino ; ou
(f) pour la production d'un composé de formule I dans laquelle Ar¹ porte un substituant alkyle ou alkyle substitué sur un atome d'azote disponible, dans laquelle Ar² porte un substituant alkoxy ou dans laquelle R¹ représente un groupe alkoxyalkyle, l'alkylation d'un composé de formule I dans laquelle Ar¹ porte un atome d'hydrogène sur ledit atome d'azote disponible, dans laquelle Ar² porte un substituant hydroxy ou dans laquelle R¹ représente un groupe hydroxyalkyle ; et
lorsqu'un sel pharmaceutiquement acceptable d'un composé nouveau de formule I est requis, l'obtention de ce sel par réaction dudit composé avec un acide ou une base convenable au moyen d'un mode opératoire classique.

10. Composition pharmaceutique qui comprend un dérivé d'éther cyclique de formule I, ou un de ses sels pharmaceutiquement acceptables, suivant l'une quelconque des revendications 1 à 8 en association avec un diluant ou support pharmaceutiquement acceptable.
